# EUROPÄISCHE PATENTANMELDUNG

(11) **EP 1 127 576 A1**
(43) Veröffentlichungstag der Anmeldung: **29.08.2001**
(21) Anmeldenummer: 01103624.1
(22) Anmeldetag: 22.02.2001
(51) Int. Cl.: A61K 38/18, A61K 38/43, A61K 48/00, C12N 9/12, A61P 17/02

(54) **Verwendungen von NM23-Polypeptiden bei Haut- und Darmerkrankungen**

(30) Priorität: 23.02.2000 DE 10008330; 24.04.2000 US 199312 P
(71) Anmelder: Switch Biotech Aktiengesellschaft, 82152 Martinsried (DE); ETH Zürich, ETH Zentrum/ Hönggerberg, 8092 Zürich (CH)
(72) Erfinder: Werner, Sabine, 8032 Zürich (CH); Braun, Susanne, 8050 Zürich (CH); Halle, Jörn-Peter, 82377 Penzberg (DE); Goppelt, Andreas, 80636 München (DE); Regenbogen, Johannes, 82152 Martinsried (DE)
(74) Vertreter: Bösl, Raphael, Dr. rer. nat., Dipl.-Chem.

(57) **Zusammenfassung**

Verwendung von Polypeptiden oder diese kodierende Nukleinsäuren der Genfamilie NM23 zur Diagnose und/oder Behandlung von Erkrankungen von Haut- und/oder Darmzellen und/oder von Wundheilung und/oder deren krankhaften Störungen sowie ihre Verwendung zur Identifizierung von pharmakologisch aktiven Substanzen.

## Beschreibung

Die Erfindung betrifft die Verwendung von Polypeptiden oder diese kodierende Nukleinsäuren der Genfamilie NM23 zur Diagnose und/oder Prävention und/oder Behandlung von Erkrankungen von Haut- und/oder Darmzellen und/oder von Wundheilung und/oder deren krankhaften Störungen sowie ihre Verwendung zur Identifizierung von pharmakologisch aktiven Substanzen.

Wunden heilen im allgemeinen ohne therapeutischen Eingriff ab. Es gibt jedoch zahlreiche Erkrankungen, bei denen die Wundheilung krankhaft gestört ist, wie z.B. Diabetes mellitus, arterielle Verschlußkrankheiten, Schuppenflechte (Psoriasis), atopische Dermatitis, Kontaktdermatitis, Morbus Crohn, Epidermolysis bullosa, altersbedingte Hautveränderungen oder Innervationsstörungen. Wundheilungsstörungen führen zu einer verzögerten Wundheilung oder zu chronischen Wunden. Diese Störungen können durch die Art der Verwundung (z.B. großflächige Wunden, tiefgehende und mechanisch gedehnte Operationswunden, Verbrennungen, Trauma, Dekubitus), medikamentöse Behandlung der Patienten (z.B. mit Corticoiden) aber auch durch die Art der Erkrankung selber verursacht werden. So leiden z.B. 25% der Patienten mit Typ-II-Diabetes häufig an chronischen Ulzera ("diabetischer Fuß"), von denen etwa die Hälfte aufwendige stationäre Behandlungen erfordern und letztlich doch schlecht heilen. Der diabetische Fuß verursacht mehr Klinikaufenthalte als jede andere mit Diabetes assoziierte Komplikation. Die Zahl dieser Fälle bei Diabetes Typ I und II ist im Steigen begriffen und repräsentiert ca. 2,5% aller Klinikeinweisungen. Zudem heilen Wunden mit zunehmendem Alter der Patienten schlechter. Oft ist auch eine Beschleunigung des natürlichen Wundheilungsprozesses wünschenswert, um z.B. die Gefahr von bakteriellen Infektionen oder die Liegezeiten der Patienten zu verringern.

Auch nach erfolgtem Wundverschluß kann es zu weiteren Störungen kommen. Während Wunden fötaler Haut ohne Narbenbildung heilen, kommt es nach Verletzungen in der Postnatalperiode stets zu Narbenbildungen, die oft ein großes kosmetisches Problem darstellen. Bei Patienten mit großflächigen Brandwunden kann zudem die Lebensqualität dramatisch beeinträchtigt werden, zumal bei vernarbter Haut auch die Anhangsgebilde, wie Haarfollikel, Schweiß- und Talgdrüsen fehlen. Bei entsprechender genetischer Disposition kann es auch zu Keloiden kommen, hypertrophischen Narben, die in die umgebende Haut einwuchern.

Der Vorgang der Wundheilung erfordert komplexe koordiniert ablaufende Wirkungen und Interaktionen verschiedener Zelltypen. Im Wundheilungsprozeß unterscheidet man folgende Schritte: die Blutgerinnung im Bereich der Wunde, die Rekrutierung von Entzündungszellen, die Reepithelialisierung, die Bildung von Granulationsgewebe sowie die Restrukturierung von Matrix. Die exakten Reaktionsmuster der beteiligten Zelltypen während der Phasen der Proliferation, der Migration, der Matrixsynthese und der Kontraktion sind, ebenso wie die Regulation von Genen wie z.B. Wachstumsfaktoren, Rezeptoren und Matrixproteinen, bislang wenig bekannt.

So sind bisher nur wenig zufriedenstellende Therapien entwickelt worden, um bei Wundheilungsstörungen eingreifen zu können. Etablierte Therapieformen beschränken sich auf physikalische Unterstützung der Wundheilung (z.B. Verbände, Kompressen, Gele) oder der Transplantation von Hautgeweben, gezüchteten Hautzellen und/oder Matrixproteinen. In den letzten Jahren sind Wachstumsfaktoren zur Verbesserung der Wundheilung erprobt worden, ohne jedoch die konventionelle Therapie entscheidend zu verbessern. Auch die Diagnose von Wundheilungsstörungen beruht auf wenig aussagekräftigen optischen Analysen der Haut, da ein tieferes Verständnis der Genregulation während der Wundheilung bisher fehlt.

Auch für andere Störungen von regenerativen Prozessen sind bisher wenig zufriedenstellende Therapien entwickelt worden. Auch hier ist die Kenntnis der Genregulation für die Entwicklung von Diagnostika und Therapien vorteilhaft. Es ist gezeigt worden (Finch et al., 1997, Am. J. Pathol. 151: 1619-28; Werner, 1998, Cytokine Growth Factor Rev. 9: 153-165), daß wundheilungsrelevante Gene auch bei dermatologischen Erkrankungen, die auf Störungen der Regeneration der Haut beruhen, und allgemein bei regenerativen Prozessen eine entscheidende Rolle spielen. So spielt der Wachstumsfaktor KGF nicht nur eine entscheidende Rolle bei der Regulation der Proliferation und Differenzierung von Keratinozyten während der Wundheilung, sondern ist auch ein wichtiger Faktor bei der Hyperproliferation der Keratinozyten bei der Schuppenflechte (Psoriasis) und Regenerationsprozessen im Darm (bei Morbus Crohn und Colitis Ulcerosa)

Es ist daher Aufgabe der vorliegenden Erfindung, Polypeptide und/oder diese kodierende Nukleinsäuren zur Verfügung zu stellen, die an Prozessen bei Erkrankungen von Haut- und/oder Darmzellen und/oder bei der Wundheilung und/oder deren krankhaften Störungen beteiligt sind, und deren Verwendung die Diagnose und/oder Prävention und/oder Behandlung sowie die Identifizierung und Entwicklung von im Zusammenhang mit diesen Erkrankungen wirksamen Pharmazeutika und Diagnostika entscheidend verbessert.

Die Erkrankungen der Haut und des Darms, die Wundheilung und deren krankhafte Störungen im Sinne der Erfindung sind abzugrenzen von Haut- und Darmerkrankungen, die mit unkontrolliertem Gewebewachstum und Zelldifferenzierung einhergehen, insbesondere von Haut- und Darmkrebs. Bei letzteren kommt es zur Transformation einzelner Zellen, die dadurch unkontrolliert, autonom, d.h. von Interaktionen mit anderen Zelltypen isoliert zu proliferieren beginnen und dabei die pathologischen Veränderungen an die Tochterzellen vererben. Es handelt sich also um eine Erkrankung, die mit einem Verlust von Interaktionen, beispielsweise von Zell-Zell Adhäsion und von typischen Zelleigenschaften einhergeht. Hingegen beruhen Krankheiten im Sinne der Erfindung auf Störungen der Zell-Zell Interaktionen. Die Entstehung von Hauterkrankungen im Sinne der Erfindung, sind durch eine Vielzahl von Faktoren bedingt. So spielen z.B. im Falle der Psoriasis, wahrscheinlich sowohl genetische Prädispositionen als auch Fehlfunktionen der T-Zellen, Fibroblasten und Keratinozyten eine Rolle (siehe z.B. Nair et al., 1997; Hum. Molec. Genet. 6: 1349-1356; Gottlieb et al., 1995, Nat. Med. 1: 442-447; Saiag et al., 1985, Science, 230: 669-672; Pittelkow, 1998, in Roenigk 1998: 225-246). Auch der Verlauf der Wundheilung kann durch verschiedenste endogene und exogene Faktoren moduliert sein. Bereits kleine Störungen der Interaktionen zwischen den unterschiedlichen Zelltypen der Dermis und Epidermis selbst, aber auch Wechselwirkungen mit anderen Geweben und Organen wie dem Blutgefäßsystem, dem Nervensystem und dem Bindegewebe, können zu gestörter Wundheilung gefolgt von Narbenbildung führen. Weiterhin können Infektionen, Alterung, Erkrankungen, wie Diabetes und Immunerkrankungen sowie Vitaminmängel den Wundheilungsprozeß beeinträchtigen. Ähnlich komplexe Wechselwirkungen sind auch für andere Hauterkrankungen wie Vitiligo und atopische Dermatitis beschrieben. Dies unterscheidet die Hauterkrankungen wesentlich von Krebserkrankungen dieser Organe.

Der autonome Charakter der Krebserkrankungen zeigt sich auch auf therapeutischer Ebene. Im Falle nicht-metastasierender Tumore läßt sich Krebs chirurgisch behandeln. Diese physikalische Behandlungsmöglichkeit ist möglich, da zwischen Tumorzellen und den umliegenden Zellen und Geweben keine Interaktionen stattfinden, so das durch einfache Exzision des Tumors der Patient geheilt werden kann, während dies bei Hautkrankheiten im Sinne der Erfindung nicht möglich ist - die krankhaften Störungen der Zell-Zell und/oder Gewebe-Gewebe Interaktionen können nicht durch Herausschneiden betroffener Hautstellen behoben werden.

Daß es sich bei den verglichenen Krankheiten um Krankheiten handelt, denen ein grundsätzlich unterschiedlicher Mechanismus zugrunde liegt, wird deutlich, wenn man die therapeutischen Ansätze vergleicht. Bei Krebserkrankungen und Krankheiten, die mit unkontrollierter Zellproliferation einhergehen, ist die Therapie auf die Abtötung schnell wachsender Zellen z.B. mittels Cytostatika gerichtet. Diese toxischen Substanzen verhindern das Wachstum aktiv proliferierender Zellen während Zellen in der G0-Phase des Zellzyklus nicht beeinflußt werden. Hingegen zielt die Behandlung von Erkrankungen von Hautzellen im Sinne der Erfindung auf die Modulation der Interaktionen zwischen den verschiedenen Zelltypen, wie z.B. durch die Beeinflussung der Migration, Proliferation und Differenzierung *einzelner* Zelltypen. Hauterkrankungen im Sinne der Erfindung können nicht durch generelle Inaktiiverung proliferierender Zellen geheilt werden.

Der methodische Ansatz zur Identifizierung der erfindungsgemäß verwendeten Nukleinsäuren, die an der Wundheilung und/ oder an Prozessen der Haut-/ Darmerkrankungen im Sinne der Erfindung beteiligt sind unterscheidet sich deutlich von Verfahren, die geeignet sind Nukleinsäuren zu identifizieren, die an Prozessen der Krebserkrankungen beteiligt sind. Letztere können durch Analyse differentiell exprimierter Gene des vom Krebs betroffenen Zelltyps identifiziert werden. Ziel des Assays der vorliegenden Erfindung ist es jedoch vielmehr, durch Vergleich der Expression in kranken und gesunden Gewebebiopsien Gene zu identifizieren, die an den komplexen Prozesse der Hauterkrankungen und/oder an der Wundheilung und/oder deren krankhaften Störungen beteiligt sind. Zur Identifizierung von krebsrelevanten Gene wäre dieses Verfahren ungeeignet.

In Analogie zur Unterscheidung der Hauterkrankungen im Sinne der Erfindung von Hautkrebs und Hauterkrankungen, die mit unkontrolliertem Gewebewachstum und Zelldifferenzierung einhergehen, lassen sich auch Darmerkrankungen im Sinne der Erfindung von Darmkrebs und Erkrankungen, die mit unkontrolliertem Gewebewachstum und Zelldifferenzierung einhergehen, abgrenzen. So werden schlecht heilende Ulzera des Dickdarms, beispielsweise Morbus Crohn, wie bereits für die Haut dargelegt, durch verschiedene Faktoren hervorgerufen und moduliert, die über Störungen der Zell-Zell-Interaktionen wirken. Autoimmun-Mechanismen, Cytokin Polymorphismen, Bakterien und infektiöse Agentien sind Beispiele für diese Faktoren (Perner und Rask-Madsen, 1999, Aliment. Pharmacol. Ther. 13:135-144). Diese Faktoren stören die Interaktionen zwischen den Zellen des Darmepithels, wie den Zellen der Krypten, den Villizellen oder den Phagozyten (Ruemmele und Seidman, 1998, Chung Hua Min Kuo Hsiao Erh Ko I Hsueh Hui Tsa Chih, 39:1-8).

Die Rasteruntersuchung (Screen) im Sinne der Erfindung ist daher auch nicht geeignet Nukleinsäuren zu identifizieren, die an Darmkrebs-spezifischen Vorgängen beteiligt sind, während das Verfahren sehr leistungsfähig im Zusammenhang mit Darmerkrankungen ist.

Bei der Analyse der Genexpression während des Wundheilungsprozesses sowie bei Psoriasis und bei Morbus Crohn konnte die Genfamilie NM23 identifiziert werden, deren bereits bekannte und beschriebene Funktionen bisher nicht mit Haut und/oder Darmerkrankungen, beispielsweise bei einer gestörten Wundheilung, in Verbindung gebracht wurden, deren Regulation aber für den Heilungsprozeß essentiell ist und die damit erstmals in kausalen Zusammenhang mit der Diagnose und/oder Prävention und/oder Behandlung von Haut- und/oder Darmerkrankungen, beispielsweise bei einer gestörten Wundheilung, gebracht wurden. Die Polypeptide dieser Genfamilie gehören nicht zu den bisher bekannten geeigneten Zielen für die Diagnose - wie beispielsweise der Indikation - und/oder Prävention- und/oder Behandlung - wie beispielsweise der Modulation - von Haut- und/oder Darmerkrankungen und/oder der Wundheilung oder für die Identifizierung von pharmakologisch aktiven Substanzen, so daß sich aus dieser Erfindung völlig neue Therapieansätze und Diagnostika vergeben.

Die Aufgabe der Erfindung wird durch die Verwendung mindestens eines Polypeptids der Genfamilie NM23 gemäß einer der SEQ ID Nr. 1 bis SEQ ID Nr. 10 oder einer funktionellen Variante davon oder eine diese kodierende Nukleinsäure, oder einer Variante davon, und unten ebenfalls erläutert zur Diagnose und/oder Behandlung, beispielsweise zur therapeutischen und/oder prophylaktischen Behandlung, von Haut- und/oder Darmerkrankungen und/oder von Wundheilung und/oder deren krankhaften Störungen und/oder zur Identifizierung von pharmakologisch aktiven Substanzen gelöst.

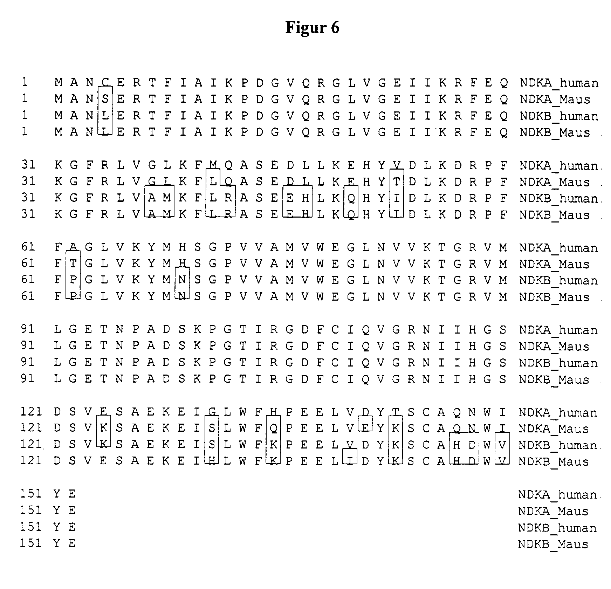

Die Genfamilie NM23 kodiert für Proteine mit Nukleotid-Diphospat-Kinase (NDK)-Aktivität (Review in Postel, 1998, Int. J. Biochem. Cell. Biol. 30:1291-5), die substratunspezifisch Nukleosid-Diphosphate in Nukleosid-Triphosphate umwandeln. Das aktive Enzym besteht aus 6 Untereinheiten der sehr homologen Polypeptide NM23A (auch als NDKA benannt, siehe Figur 6) und NM23B (NDKB), wobei alle 6 möglichen Kombinationen der Untereinheiten vorkommen können (Gilles et al., 1991 , J. Biol. Chem. 266:8784-9). Die Gene NM23-H1 (aus dem Menschen, EMBL Datenbank Einträge X17620, X75598, X73066; Rosengard et al., 1989, Nature 342:177-180) bzw. NM23-M1 (aus der Maus, EMBL M35970, M65037, U85511, AF033377; Rosengard et al., supra; Steeg et al., 1988, J. Natl. Cancer Inst. 80:200-204) kodieren für das Polypeptid NM23A/NDKA (SWISSPROT Datenbank Einträge P15531 und P15532) und die Gene NM23-H2 (EMBL X58965, M36981, L16785; Gilles et al., 1991, J. Biol. Chem. 266:8784-9; Stahl et al., 1991, Cancer Res. 51:445-9) und NM23-M2 (EMBL X68193; Urano et al., 1992, FEBS Lett. 309:358-362) für NM23B/NDKB (SWISSPROT Einträge P22392 und Q01768).

Zudem gehören zu der NM23 Genfamilie die Gene NM23-H4/NDKM (SWISSPROT Eintrag O00746; Milon et al., 1997, Hum. Genet., 99:550-557), DR-NM23/NDK3 (SWISSPROT Eintrag Q13232; Cucco et al., 1995, Proc. Natl. Acad. Sci. U.S.A., 92:7435-7439), NM23-H5/NDK5 (SWISSPROT Eintrag P56597, Munier et al., 1998, FEBS Lett., 434:289-294), Typ 5 NM23 (EMBL Eintrag U90449; Nakamura et al., 1997, direkter Eintrag in die Datenbank) und NDK6 (SWISSPROT Eintrag O60361, Bradshaw und Ozersky, 1998, direkter Eintrag in die Datenbank). Die Polypeptide der NM23 Genfamilie zeigen untereinander eine ca. 55 bis 95%-ige Identität auf dem Niveau der Aminosäuresequenz.

Neben der Funktion als Stoffwechselenzym sind der NM23 Genfamilie verschiedene andere Funktionen zugeordnet worden. So sind die Genprodukte von NM23B nicht nur im Zytoplasma, sondern auch im Zellkern lokalisiert (Kraeft et al., 1996, Exp. Cell Res. 227:63-9). Auch ist eine DNA-bindende sowie eine die Transkription aktivierende Funktion beschrieben worden (Postel et al., 1993, Science 261:478-80; Postel, 1999, J. Biol. Chem 274:22821-9). Weiterhin ist eine Funktion von NM23 bei der Regulation von Ras-GTPasen beschrieben worden (Zhu et al., Proc. Nat. Acad. Sci. USA 96:14911-8).

Die verminderte Expressionsstärke von NM23A ist als Tumormarker, für die Metastasenbildung und Zellaberrationen in *Drosophila melanogaster* beschrieben worden (Rosengard et al., 1989, Nature 342:177-180), wobei eine normale Expressionsstärke von NM23 die Fähigkeit von Tumorzellen, Metastasen zu bilden, inhibieren kann (Lee und Lee, 1999, Cancer Letter 145:93-9). Diese Funktion scheint nicht mit der Enzymaktivität verknüpft zu sein (Lee und Lee, supra). Andererseits ist die Expression von NM23 für die Zellproliferation erforderlich, wie Antisense- (Cipollini et al., 1997, Int. J. Cancer 73:297-302) und Antikörper-Experimente (Sorscher et al., 1993, Biochem. Biophys. Res. Commun. 195:336-45) vermuten lassen. Weitere Analysen der Expression von NM23 in menschlichen frühen und metastasierenden Melanomzellen sowie verschiedenen Stadien der Melanombildung führten jedoch zu dem Ergebnis, daß die Expression von NM23 im Menschen im Gegensatz zur Maus nicht mit der Metastasierung korrelierte (Easty et al.; 1996, Br. J. Cancer 74(1): 109-14). Diese Ergebnisse konnten bei einer zweiten Untersuchung von einer anderen Gruppe bestätigt werden (Seregard et al., 1999, Exp. Eye Res. 69(6): 671-676). NM23 ist somit für eine zuverlässige Diagnose bei der Melanombildung ungeeignet.

Aufgrund der weiter oben ausgeführten stark divergierenden Therapieansätze bei Erkrankungen im Sinne der Erfindung und bei Krebserkrankungen, ist es daher keine erfolgversprechende Strategie, NM23 Polypeptide und/oder deren funktionelle Varianten und/oder diese kodierende Nukleinsäuren, für die Behandlung, Diagnose von Krankheiten im Sinne der Erfindung zu verwenden, wie dies für Krebstherapie und Erkrankungen, die mit unkontrolliertem Gewebewachstum und Zelldifferenzierung einhergehen, beschrieben worden ist (WO 98/11232). Zudem wurde für keines der Polypeptide der Genfamilie NM23, diese kodierende Nukleinsäuren oder cDNAs bisher ein Zusammenhang mit Haut- oder Darmkrankheiten oder mit der Wundheilung oder deren krankhaften Störungen beschrieben oder nahegelegt. Es war daher unerwartet, daß diese Nukleinsäuren und/oder Polypeptide erfindungsgemäß verwendet werden können.

Allgemein ist die Analyse von differentiell exprimierten Genen in Geweben mit deutlich mehr Fehlern in Form von falsch positiven Klone behaftet, als die Analyse von Zellkultursystemen. Dieses Problem kann nicht durch die Verwendung eines definierten Zellkultursystems umgangen werden, da bestehende, einfache Zellkultursysteme die Komplexität der Wundheilungsprozesse im Gewebe nicht ausreichend simulieren können.

Das Problem besteht insbesondere bei der Haut, die aus einer Vielzahl von verschiedenen Zelltypen besteht. Darüber hinaus ist der Prozeß der Wundheilung ein höchst komplizierter Vorgang, der zeitliche und räumliche Änderungen zellulärer Vorgänge, wie Proliferation und Differenzierung bei den unterschiedlichen Zelltypen umfaßt. Der Ansatz, nicht nur das komplexe Zellsystem Haut, sondern darüber hinaus den physiologischen Prozeß der Wundheilung und sogar verschiedenste Wundheilungsstadien auf der Ebene differentiell exprimierter Gene zu untersuchen ist daher für einen Fachmann keine erfolgversprechende Strategie. Der Erfolg der Rasteruntersuchung war aufgrund dieser Schwierigkeiten wesentlich von der Wahl der experimentellen Parameter abhängig. Während die verwendeten Methoden (z.B. subtraktiver Hybridisierung) Standardmethoden sind, ist die Rasteruntersuchungs- und Verifizierungsstrategie durch die durchdachte und definierte Wahl von Parametern an sich bereits erfinderisch. Beispielsweise ist der Zeitpunkt der Biopsienahme kritisch für den Erfolg der Rasteruntersuchung: Wundheilungsstörungen und Hautkrankheiten liegen häufig Störungen bei der Zellproliferation und Zellmigration zugrunde. Diese Prozesse werden einen Tag nach der Verwundung initiiert, weshalb eine Analyse der molekularen Prozesse vor diesem Zeitpunkt wenig Aufschluß über die Vorgänge liefern würde, die für eine normal verlaufende Wundheilung essentiell sind. Andererseits verändert sich im Verlauf der Wundheilung später als einen Tag nach der Verwundung die Zusammensetzung der Zelltypen in der Wunde stark. Dies kann dazu führen, daß eine differentielle Expression eines bestimmten Gens in der Wunde gemessen wird, die nicht auf veränderter Expression in den Zellen beruht, sondern nur auf der unterschiedlichen Zellzusammensetzung. Dies verdeutlicht, daß die Wahl des Tages der Biopsienahme entscheidend den Erfolg der Rasteruntersuchung beeinflußte.

Trotz der definierten Parameter wurde eine Überrepräsentation von Genen beobachtet, die während der Wundheilung differentiell exprimiert werden, die aber für die Verwendung in der Wundheilung oder bei Hautkrankheiten ungeeignet sind. Diese Gene umfassen beispielsweise Gene, die für Enzyme des Primärstoffwechsels, wie Glykolyse, Citratzyklus, Glukoneogenese und Atmungskette kodieren, aber auch Gene, die für ribosomale Proteine kodieren, z.B. L41 und S20. Nur eine vergleichsweise kleine Zahl an Genen konnte als geeignet identifiziert werden. Daher war eine Identifizierung der erfindungsgemäß verwendbaren Gene als wundheilungsrelevante Gene überraschend.

Zudem gibt es enorme Variabilitäten des Wundzustands zum Zeitpunkt einer möglichen Biopsie des Patienten beim Erstkontakt mit dem Arzt. Daher wurde zur Identifikation der vorangehend beschriebenen Nukleinsäuren ein Tiermodell verwendet. Es wurden BALB/c Mäuse verwundet und zu verschiedenen Zeitpunkten Wundbiopsien entnommen. Dieses Verfahren hat den Vorteil, das sich die Randbedingungen wie genetischer Hintergrund, Art der Wunde, Zeitpunkt der Biopsie etc. exakt kontrollieren lassen und so erst eine reproduzierbare Analyse der Genexpression erlauben. Selbst unter den definierten Maus-Bedingungen ergeben sich weitere methodische Probleme wie Redundanz der analysierten Klone und Unterrepräsentation von schwach exprimierten Genen, die die Identifikation von relevanten Genen erschweren.

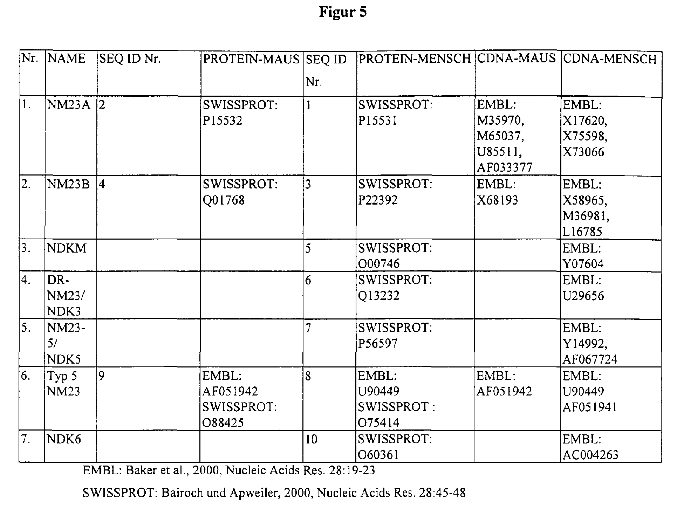

Die Zugangsnummern der erfindungsgemäß verwendbaren Polypeptide der Genfamilie NM23 und ihrer cDNAs sind in Figur 5 aufgeführt. Die cDNA des erfindungsgemäß verwendbaren Polypeptids NM23-M2 wurde aus cDNA Bibliotheken isoliert, die aus intakter und verwundeter Haut gewonnen wurden. Dabei wurden die cDNAs ausgewählt, die unterschiedliche Häufigkeiten in den cDNA-Bibliotheken der gut heilenden im Vergleich zu schlecht heilenden Wunden aufwiesen (Beispiel 1). Dies geschah beispielsweise mit Hilfe von subtraktiver Hybridisierung (Diatchenko et al., 1996, Proc. Natl. Acad. Sci. USA 93: 6025-30). Die so ausgewählte cDNA ist im schlecht heilenden cDNA Pool von Tieren, die mit dem Glukokortikoid Dexamethason behandelt wurden, deutlich häufiger vorhanden als im gut heilenden cDNA Pool.

NM23-M2 konnte durch eine subtraktive Hybridisierung von Maus-Wund-cDNA identifiziert werden. NM23-M2 war sowohl in einer cDNA Population angereichert, die aus einer Subtraktion von 1 Tages-Wunden gegen intakte Haut gewonnen wurden, als auch in einer cDNA Population, die aus der Subtraktion von schlecht heilenden (Dexamethason behandelte Mäuse) gegen gut heilende Wunden gewonnen wurden (Beispiel 1). Dies bedeutet, daß NM23 nicht nur während der normalen Wundheilung reguliert ist, sondern daß die Regulation der Expression essentiell für eine normal verlaufende Wundheilung ist.

Nach der primären Identifikation eines Gens ist es notwendig, die wundheilungsspezifische Expression durch eine weitere Methode zu bestätigen. Dies erfolgte mit Hilfe von sogenannten "Reverse Northern Blots", "RNase Protection Assays", "RT-PCR Assays" , "in situ Hybridisierung" und "TaqMan Analyse". Mit diesen Methoden wurde die Menge an mRNA in Geweben aus verschiedenen Wundheilungszuständen und in Hauterkrankungen (Psoriasis) und Darmerkrankungen (Morbus Crohn) bestimmt bzw. hauterkrankungsspezifische lokale Änderungen des Expressionsmusters in Biopsien nachgewiesen (Beispiele 2 bis 7).

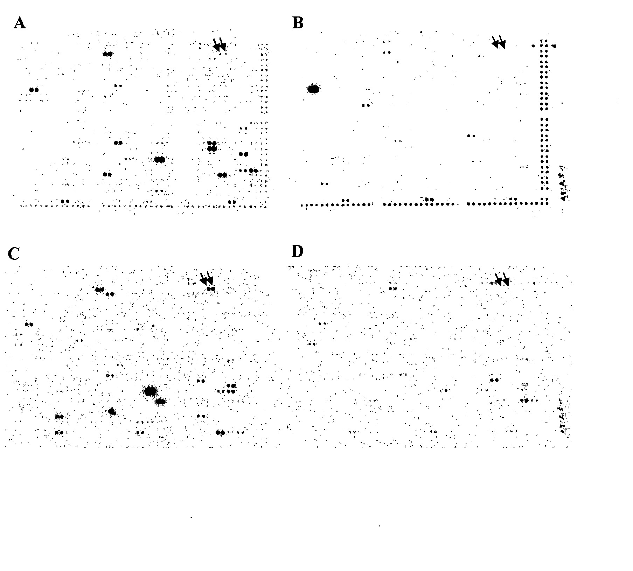

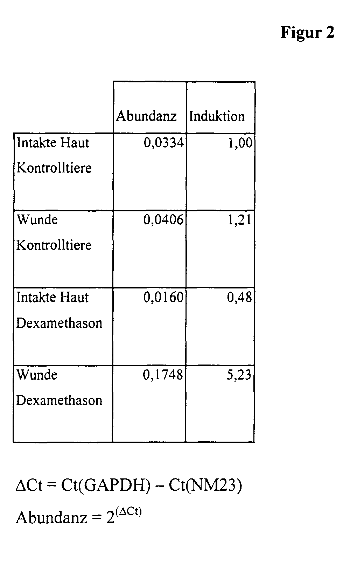

Im reversed Northern Blot konnte die Anreicherung der NM23-M2 cDNA nach Subtraktion bestätigt werden (Figur 1, Beispiel 1). Zudem zeigte sich durch in situ Hybridisierung an Gewebeschnitten von 5 Tages-Wunden von Mäusen, daß das Gen NM23-M1 stark im hyperproliferativen Epithel am Rand des Wundgewebes exprimiert wird, was für eine entscheidende Rolle bei der Proliferation von Keratinozyten und der Reepithelialisierung der Wunde spricht (Beispiel 4). Auch ergab sich durch quantitative RT-PCR Analysen, daß die Expression von NM23-M2 in schlecht heilenden Wunden von Dexamethason behandelten Mäusen ca. 5-fach stärker war, als in normal gut heilenden Wunden von Kontrolltieren (Figur 2, Beispiel 2). So konnte die durch die Subtraktionsexperimente gefundene essentielle Rolle von NM23 am Wundheilungsprozess bestätigt werden.

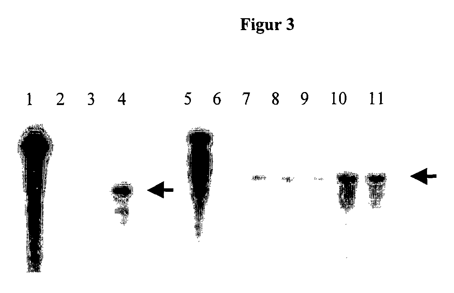

Weiterhin konnte die Expression von NM23 mit Psoriasis in Zusammenhang gebracht werden. Es wurde ein RNase Protection Assay mit RNA durchgeführt, die aus Biopsien von läsionalen Hautpartien von Psoriasispatienten bzw. aus Biopsien von Kontrollpersonen mit normaler Haut gewonnen war. Dabei zeigte sich, daß NM23-H1 in der Haut der Patienten deutlich stärker exprimiert wurde als in der Haut von Kontrollpersonen (Figur 3, Beispiel 3). Es ergibt sich also auch für die Psoriasis ein Zusammenhang zwischen NM23-Expression und Krankheitsverlauf.

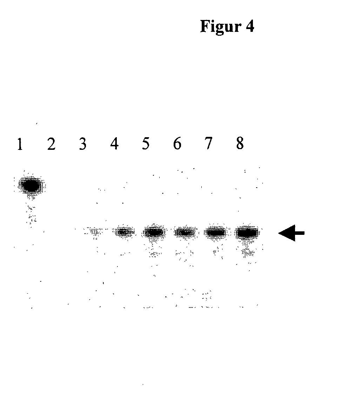

Eine ähnliche Korrelation ergab sich bei der entzündlichen Darmerkrankung Morbus Crohn. Hier wurden den Patienten Darmbiopsien von deutlich und/oder wenig entzündlichen Arealen entnommen. Im Vergleich zu einer Darmbiopsie von einer gesunden Kontrollperson zeigten alle Darmbiopsien der Morbus Crohn Patienten eine signifikant stärkere Expression von NM23-H1 (Figur 4, Beispiel 3). Zudem ergab sich eine Korrelation der NM23-H1 Expression mit der Stärke der Erkrankung. Während wenig entzündliche Areale des Darms nur eine moderate Erhöhung der Expression von NM23-H1 zeigten, ergab sich für deutlich entzündete Areale eine starke Erhöhung der Expression (Figur 4). Diese Befunde zeigten, daß die Expression von NM23 nicht nur das Ausmaß der Krankheit reflektiert, sondern essentiell für den Krankheitsverlauf ist und daß die Expression von NM23 als diagnostischer Marker genutzt werden kann.

Die erfindungsgemäß verwendbaren Polypeptide können weiterhin dadurch gekennzeichnet sein, daß diese synthetisch hergestellt werden. So kann das gesamte Polypeptid oder Teile davon zum Beispiel mit Hilfe der klassischen Synthese (Merrifield-Technik) synthetisiert werden. Teile der vorangehend beschriebenen Polypeptide eignen sich insbesondere zur Gewinnung von Antiseren, mit deren Hilfe geeignete Genexpressionsbanken durchsucht werden können, um so zu weiteren funktionellen Varianten eines erfindungsgemäß verwendbaren Polypeptids zu gelangen.

Unter dem Begriff "funktionelle Varianten" im Sinne der vorliegenden Erfindung versteht man Polypeptide, die funktionell mit den erfindungsgemäß verwendbaren Polypeptiden verwandt sind, d. h. während regenerativer Prozesse der Haut oder des Darms reguliert werden und/oder Strukturmerkmale der Polypeptide aufweisen. Beispiele funktioneller Varianten sind Polypeptide, die durch unterschiedliche Allele des Gens, in verschiedenen Individuen oder in verschiedenen Organen eines Organismus kodiert werden.

Zu funktionellen Varianten zählen beispielsweise auch Polypeptide, die von Nukleinsäuren kodiert werden, die aus nicht-haut- oder nicht-darm-spezifischem Gewebe, z. B. Embryonalgewebe, isoliert werden, jedoch nach Expression in einer an der Wundheilung beteiligten Zelle die bezeichneten Funktionen besitzen.

Im weiteren Sinne versteht man darunter auch Polypeptide, die eine Sequenzhomologie, insbesondere eine Sequenzidentität, von ca. 70%, vorzugsweise ca. 80%, insbesondere ca. 90%, vor allem ca. 95% zu dem Polypeptid mit der Aminosäuresequenz gemäß einer der SEQ ID Nr. 1 bis SEQ ID Nr. 10 und/oder zu den DNA-Sequenzen der öffentlich zugänglichen Datenbankeinträge der Liste in der Figur 5 aufweisen. Funktionelle Varianten des Polypeptids können auch Teile des erfindungsgemäß verwendeten Polypeptids mit mindestens 6 Aminosäuren Länge, vorzugsweise mit mindestens 8 Aminosäure Länge, insbesondere mit mindestens 12 Aminosäure Länge sein. Umfaßt sind auch N- und/oder C-terminale und/oder interne Deletionen des Polypeptids im Bereich von ca. 1-60, vorzugsweise von ca. 1-30, insbesondere von ca. 1-15, vor allem von ca. 1-5 Aminosäuren. Beispielsweise kann die erste Aminosäure Methionin fehlen, ohne daß die Funktion des Polypeptids wesentlich verändert wird.

Der Begriff "kodierende Nukleinsäure" bezieht sich auf eine DNA-Sequenz, die für ein isolierbares erfindungsgemäß verwendbares Polypeptid oder einen Vorläufer, z.B. mit einer Signalsequenz, kodiert. Das Polypeptid kann durch eine Sequenz in voller Länge oder jeden Teil der kodierenden Sequenz kodiert werden, solange die spezifische, beispielsweise enzymatische Aktivität, erhalten bleibt.

Es ist bekannt, daß Veränderungen in der Sequenz der erfindungsgemäß verwendbaren Nukleinsäuren vorhanden sein können, zum Beispiel durch die Degenerierung des genetischen Codes, oder daß nicht translatierte Sequenzen beispielsweise am 5'-und/oder 3'-Ende der Nukleinsäure angehängt sein können, ohne daß dessen Aktivität wesentlich verändert wird. Auch können die unten näher beschriebenen Modifikationen durchgeführt werden. Diese Erfindung umfaßt deshalb auch sogenannte "Varianten" der vorangehend beschriebenen Nukleinsäuren.

Mit dem Begriff "Varianten" sind alle DNA-Sequenzen bezeichnet, die komplementär zu einer DNA-Sequenz sind, die unter stringenten Bedingungen mit der Referenzsequenz hybridisieren und für ein Polypeptid kodieren, das eine im wesentlichen gleiche Aktivität aufweist, wie das von der Referenzsequenz kodierte Polypeptid.

Unter dem Begriff "Regulation" wird beispielsweise die Erhöhung oder Erniedrigung der Polypeptidmenge oder diese kodierende Nukleinsäure verstanden, wobei diese Änderung beispielsweise auf transkriptioneller oder translationeller Ebene stattfinden kann.

Unter "stringenten Hybridisierungsbedingungen" sind solche Bedingungen zu verstehen, bei denen eine Hybridisierung beispielsweise bei 60°C in 2,5 x SSC-Puffer, gefolgt von mehreren Waschschritten bei 37°C in einer geringeren Pufferkonzentration erfolgt und stabil bleibt.

Varianten der Nukleinsäuren können auch Teile der erfindungsgemäß verwendeten Nukleinsäuren mit mindestens 8 Nukleotiden Länge, vorzugsweise mit mindestens 18 Nukleotiden Länge, insbesondere mit mindestens 24 Nukleotiden Länge, besonders bevorzugt mit mindestens 30 Nukleotiden, vorzugsweise bevorzugt mit mindestens 42 Nukleotiden sein.

Bevorzugterweise handelt es sich bei den erfindungsgemäß verwendeten Nukleinsäuren um DNA oder RNA, vorzugsweise eine DNA, insbesondere eine doppelsträngige DNA. Weiterhin kann die Sequenz der Nukleinsäuren dadurch gekennzeichnet sein, daß sie mindestens ein Intron und/oder eine polyA-Sequenz aufweist. Die erfindungsgemäß verwendbaren Nukleinsäuren können auch in Form ihrer antisense-Sequenz vorliegen.

Für die Expression des betreffenden Gens ist im allgemeinen eine doppelsträngige DNA bevorzugt, wobei der für das Polypeptid kodierende DNA-Bereich besonders bevorzugt ist. Dieser Bereich beginnt mit dem ersten in einer Kozak Sequenz (Kozak, 1987, Nucleic. Acids Res. 15:8125-48) liegenden Start-Codon (ATG) bis zum nächsten Stop-Codon (TAG, TGA bzw. TAA), das im gleichen Leseraster zum ATG liegt.

Eine weitere Verwendung der erfindungsgemäß verwendeten Nukleinsäuresequenzen ist die Konstruktion von antisense Oligonukleotiden (Zheng und Kemeny, 1995, Clin. Exp. Immunol. 100:380-2; Nellen und Lichtenstein, 1993, Trends Biochem. Sci. 18:419-23; Stein, 1992, Leukemia 6:967-74) und/oder Ribozymen (Amarzguioui, et al. 1998, Cell. Mol. Life Sci. 54:1175-202; Vaish, et al., 1998, Nucleic Acids Res. 26:5237-42; Persidis, 1997, Nat. Biotechnol. 15:921-2; Couture und Stinchcomb, 1996, Trends Genet. 12:510-5). Mit antisense Oligonukleotiden kann man die Stabilität der vorangehend beschriebenen Nukleinsäure verringern und/oder die Translation der vorangehend beschriebenen Nukleinsäure inhibieren. So kann beispielsweise die Expression der entsprechenden Gene in Zellen sowohl *in vivo* als auch *in vitro* verringert werden. Antisense Oligonukleotide oder Ribozyme können sich daher als Therapeutikum eignen. Diese Strategie eignet sich beispielsweise auch für Haut, epidermale und dermale Zellen, insbesondere, wenn die antisense Oligonukleotide mit Liposomen komplexiert werden (Smyth et al., 1997, J. Invest. Dermatol. 108:523-6; White et al., 1999, J. Invest. Dermatol. 112:699-705; White et al., 1999, J. Invest. Dermatol. 112:887-92). Für die Verwendung als Sonde oder als "antisense" Oligonukleotid ist eine einzelsträngige DNA oder RNA bevorzugt.

Weiterhin kann zur Durchführung der Erfindung eine Nukleinsäure verwendet werden, die synthetisch hergestellt worden ist. So kann die erfindungsgemäß verwendete Nukleinsäure beispielsweise chemisch anhand der in der Figur 5 beschriebenen DNA Sequenzen und/oder anhand der in diesen Figuren ebenfalls beschriebenen Proteinsequenzen unter Heranziehen des genetischen Codes z. B. nach der Phosphotriester-Methode synthetisiert werden (siehe z. B. Uhlmann, E. & Peyman, A. (1990) Chemical Revievs, 90, 543-584, No. 4).

Oligonukleotide werden in der Regel schnell durch Endo- oder Exonukleasen, insbesondere durch in der Zelle vorkommende DNasen und RNasen, abgebaut. Deshalb ist es vorteilhaft, die Nukleinsäure zu modifizieren, um sie gegen den Abbau zu stabilisieren, so daß über einen langen Zeitraum eine hohe Konzentration der Nukleinsäure in der Zelle beibehalten wird (Beigelman et al., 1995, Nucleic Acids Res. 23:3989-94; Dudycz, 1995, WO 95/11910; Macadam et al., 1998, WO 98/37240; Reese et al., 1997, WO 97/29116). Typischerweise kann eine solche Stabilisierung durch die Einführung von einer oder mehrerer Internukleotid-Phosphorgruppen oder durch die Einführung einer oder mehrerer Nicht-Phosphor-Internukleotide, erhalten werden.

Geeignete modifizierte Internukleotide sind in Uhlmann und Peymann (1990 Chem. Rev. 90, 544) zusammengefaßt (siehe auch Beigelman et al., 1995 Nucleic Acids Res. 23: 3989-94; Dudycz, 1995, WO 95/11910; Macadam et al., 1998, WO 98/37240; Reese et al., 1997, WO 97/29116). Modifizierte Internukleotid-Phosphatreste und/oder Nicht-Phosphorbrücken in einer Nukleinsäure, die bei einer der erfindungsgemäßen Verwendungen eingesetzt werden können, enthalten zum Beispiel Methylphosphonat, Phosphorothioat, Phosphoramidat, Phosphorodithioat, Phophatester, während Nicht-Phosphor-Internukleotid-Analoge, beispielsweise Siloxanbrücken, Carbonatbrücken, Carboxymethylester, Acetamidatbrücken und/oder Thioetherbrücken enthalten. Es ist auch beabsichtigt, daß diese Modifizierung die Haltbarkeit einer pharmazeutischen Zusammensetzung, die bei einer der erfindungsgemäßen Verwendungen eingesetzt werden kann, verbessert.

In einer weiteren Ausführungsform der Erfindung werden die erfindungsgemäß verwendeten Nukleinsäuren zur Herstellung eines Vektors, vorzugsweise in Form eines shuttle Vektors, Phagemids, Cosmids, Expressionsvektors oder gentherapeutisch wirksamen Vektors zur Analyse und/oder Diagnose und/oder Prävention und/oder Behandlung von Haut- und Darmerkrankungen und/oder von Wundheilung und/oder deren krankhaften Störungen, verwendet. Weiterhin können unter Verwendung der vorangehend beschriebenen Nukleinsäuren in Form von knock-out Genkonstrukten oder Expressionskassetten verwendet werden.

So kann die erfindungsgemäß verwendete Nukleinsäure in einem Vektor vorzugsweise in einem Expressionsvektor oder gentherapeutisch wirksamen Vektor enthalten sein. Vorzugsweise enthält der gentherapeutisch wirksame Vektor wund-, darm- bzw. hautspezifische regulatorische Sequenzen, die funktionell mit der vorangehend beschriebenen Nukleinsäure verbunden sind.

Die Expressionsvektoren können prokaryotische oder eukaryotische Expressionsvektoren sein. Beispiele für prokaryotische Expressionsvektoren sind für die Expression in *E. coli* z.B. die Vektoren pGEM oder pUC-Derivate und für eukaryotische Expressionsvektoren für die Expression in *Saccharomyces cerevisiae* z. B. die Vektoren p426Met25 oder p426GAL1 (Mumberg et al. (1994) Nucl. Acids Res., 22, 5767-5768), für die Expression in Insektenzellen z. B. *Baculovirus*-Vektoren wie in EP-B1-0 127 839 oder EP-B1-0 549 721 offenbart, und für die Expression in Säugerzellen z. B. die Vektoren Rc/CMV und Rc/RSV oder SV40-Vektoren, welche alle allgemein erhältlich sind.

Im allgemeinen enthalten die Expressionsvektoren auch für die jeweilige Wirtszelle geeignete Promotoren, wie z. B. den trp-Promotor für die Expression in *E. coli* (siehe z. B. EP-B1-0 154 133), den Met 25-, GAL 1- oder ADH2-Promotor für die Expression in Hefen (Russel et al. (1983), J. Biol. Chem. 258, 2674-2682; Mumberg, supra), den Baculovirus-Polyhedrin-Promotor, für die Expression in Insektenzellen (siehe z. 13. EP-B1-0 127 839). Für die Expression in Säugetierzellen sind beispielsweise Promotoren geeignet, die eine konstitutive, regulierbare, gewebsspezifische, zellzyklusspezifische oder metabolischspezifische Expression in eukaryotischen Zellen erlauben. Regulierbare Elemente gemäß der vorliegenden Erfindung sind Promotoren, Aktivatorsequenzen, Enhancer, Silencer und/oder Repressorsequenzen.

Beispiel für geeignete regulierbare Elemente, die konstitutive Expression in Eukaryonten ermöglichen, sind Promotoren, die von der RNA Polymerase III erkannt werden oder virale Promotoren, CMV-Enhancer, CMV-Promotor, SV40-Promotor oder LTR-Promotoren z. B. von MMTV (mouse mammary tumour virus; Lee et al. (1981) Nature 214, 228-232) und weitere virale Promotor- und Aktivatorsequenzen, abgeleitet aus beispielsweise HBV, HCV, HSV, HPV, EBV, HTLV oder HIV.

Beispiele für regulierbare Elemente, die induzierbare Expression in Eukaryonten ermöglichen, sind der Tetracyclinoperator in Kombination mit einem entsprechenden Repressor (Gossen M. et al. (1994) Curr. Opin. Biotechnol. 5, 516-20).

Vorzugsweise erfolgt die Expression von wundheilungsrelevanten Genen unter der Kontrolle von gewebespezifischen Promotoren, wobei wund-, haut- oder darmspezifische Promotoren wie beispielsweise der humane K10-Promotor (Bailleul et al., 1990. Cell 62: 697-708), der humane K14-Promotor (Vassar et al., 1989, Proc. Natl. Acad. Sci. USA 86: 1563-67), der bovine Cytokeratin IV-Promotor (Fuchs et al., 1988; The biology of wool and hair (Hrsg.: G.E. Rogers, et al.), S. 287-309. Chapman und Hall, London/New York) oder der "Fatty acid binding protein"-Promoter aus der Ratte (Erwin et al., 1999, Am. J. Physiol. 277:533-40) besonders zu bevorzugen sind.

Weitere Beispiele für regulierbare Elemente, die gewebsspezifische Expression in Eukaryonten ermöglichen, sind Promotoren oder Aktivatorsequenzen aus Promotoren oder Enhancern von solchen Genen, die für Proteine kodieren, die nur in bestimmten Zelltypen exprimiert werden.

Beispiele für regulierbare Elemente, die zellzyklusspezifische Expression in Eukaryonten ermöglichen, sind Promotoren folgender Gene: cdc25, Cyclin A, Cyclin E, cdc2, E2F, B-myb oder DHFR (Zwicker J. und Müller R. (1997) Trends Genet. 13, 3-6).

Beispiele für regulierbare Elemente, die metabolischspezifische Expression in Eukaryonten ermöglichen, sind Promotoren, die durch Hypoxie, durch Glukosemangel, durch Phosphatkonzentration oder durch Hitzeschock reguliert werden.

Um die Einführung von erfindungsgemäß verwendeten Nukleinsäuren und damit die Expression des Polypeptids in einer eu- oder prokaryotischen Zelle durch Transfektion, Transformation oder Infektion zu ermöglichen, kann die Nukleinsäure als Plasmid, als Teil eines viralen oder nicht-viralen Vektors vorliegen. Als virale Vektoren eignen sich hierbei besonders: Baculoviren, Vakziniaviren, Adenoviren, adenoassoziierte Viren und Herpesviren. Als nicht-virale Vektoren eignen sich hierbei besonders: Virosomen, Liposomen, kationische Lipide, oder poly-Lysin konjugierte DNA.

Beispiele von gentherapeutisch wirksamen Vektoren sind Virusvektoren, beispielsweise Adenovirusvektoren oder retroviralen Vektoren (Lindemann et al., 1997, Mol. Med. 3: 466-76; Springer et al., 1998, Mol. Cell. 2: 549-58). Eukaryotische Expressionsvektoren eignen sich in isolierter Form für die gentherapeutische Anwendung, da nackte DNA bei topischer Applikation in Hautzellen eindringen kann (Hengge et al., 1996, J. Clin. Invest. 97: 2911-6; Yu et al., 1999, J. Invest. Dermatol. 112: 370-5).

Gentherapeutisch wirksame Vektoren lassen sich auch dadurch erhalten, daß man die erfindungsgemäß verwendeten Nukleinsäure mit Liposomen komplexiert, da damit eine sehr hohe Transfektionseffizienz, insbesondere von Hautzellen, erreicht werden kann (Alexander und Akhurst, 1995, Hum. Mol. Genet. 4: 2279-85). Bei der Lipofektion werden kleine unilamellare Vesikel aus kationischen Lipiden durch Ultraschallbehandlung der Liposomensuspension herstellt. Die DNA wird ionisch auf der Oberfläche der Liposomen gebunden, und zwar in einem solchen Verhältnis, daß eine positive Nettoladung verbleibt und die Plasmid-DNA zu 100% von den Liposomen komplexiert wird. Neben den von Felgner et al. (1987, supra) eingesetzten Lipidmischungen DOTMA (1,2-Dioleyloxpropyl-3-trimethylammoniumbromid) und DPOE (Dioleoylphosphatidylethanolamin) wurden inzwischen zahlreiche neue Lipidformulierungen synthetisiert und auf ihre Effizienz in der Transfektion verschiedener Zellinien getestet (Behr, J.P. et al. (1989), Proc. Natl. Acad. Sci. USA 86, 6982-6986; Felgner, J.H. et al. (1994) J. Biol. Chem. 269, 2550-2561; Gao, X. & Huang, L. (1991), Biochim. Biophys. Acta 1189, 195-203). Beispiele der neuen Lipidformulierungen sind DOTAP N-[1-(2,3-Dioleoyloxy)propyl]-N,N,N-trimethylammoniumethylsulfat oder DOGS (TRANSFECTAM; Dioctadecylamidoglycylspermin). Hilfsstoffe, die den Transfer von Nukleinsäuren in die Zelle erhöhen, können beispielsweise Proteine oder Peptide, die an DNA gebunden sind oder synthetische Peptid-DNA-Moleküle, die den Transport der Nukleinsäure in den Kern der Zelle ermöglichen, sein (Schwartz et al. (1999) Gene Therapy 6, 282; Brandén et al. (1999) Nature Biotech. 17, 784). Hilfsstoffe umfassen auch Moleküle, die die Freisetzung von Nukleinsäuren in das Cytoplasma der Zelle ermöglichen (Planck et al. (1994) J. Biol. Chem. 269, 12918; Kichler et al. (1997) Bioconj. Chem. 8, 213) oder beispielsweise Liposomen (Uhlmann und Peymann (1990) supra). Eine andere besonders geeignete Form von gentherapeutischen Vektoren läßt sich dadurch erhalten, daß man die erfindungsgemäß verwendeten Nukleinsäure auf Goldpartikeln aufbringt und diese mit Hilfe der sogenannten "Gene Gun" in Gewebe, bevorzugt in die Haut, oder Zellen schießt (Wang et al., 1999, J. Invest. Dermatol., 112:775-81, Tuting et al., 1998, J. Invest. Dermatol. 111:183-8).

Eine weitere Ausführungsform eines gentherapeutisch wirksamen erfindungsgemäß verwendbaren Vektors läßt sich durch das Einbringen von "nackten" Expressionsvektoren in eine biokompatible Matrix, beispielsweise eine Kollagenmatrix, herstellen. Diese Matrix kann in Wunden eingebracht werden, um die einwandernden Zellen mit dem Expressionsvektor zu transfizieren und die erfindungsgemäß verwendeten Polypeptide in den Zellen zu exprimieren (Goldstein und Banadio, US 5,962,427).

Für die gentherapeutische Anwendung der vorangehend beschriebenen Nukleinsäure ist es auch von Vorteil, wenn der Teil der Nukleinsäure, der für das Polypeptid kodiert, ein oder mehrere nicht kodierende Sequenzen einschließlich Intronsequenzen, vorzugsweise zwischen Promotor und dem Startcodon des Polypeptids, und/oder eine polyA-Sequenz, insbesondere die natürlich vorkommende polyA-Sequenz oder eine SV40 Virus polyA-Sequenz, vor allem am 3'-Ende des Gens enthält, da hierdurch eine Stabilisierung der mRNA erreicht werden kann (Jackson, R. J. (1993) Cell 74, 9-14 und Palmiter, R. D. et al. (1991) Proc. Natl. Acad. Sci. USA 88, 478-482).

Knock-out Genkonstrukte sind dem Fachmann zum Beispiel aus den US-Patenten 5,625,122; US 5,698,765; US 5,583,278 und US 5,750,825 bekannt.

Eine weiterere Ausführungsform der vorliegenden Erfindung ist die Verwendung einer Wirtszelle, insbesondere eine Haut- oder Darmzelle, die mit einem erfindungsgemäß verwendbaren Vektor oder einem knock-out Genkonstrukt zur Analyse und/oder Diagnose und/oder Prävention und/oder Behandlung von Haut- und Darmerkrankungen und/oder von Wundheilung und/oder deren krankhaften Störungen, transformiert ist. Wirtszellen können sowohl prokaryotische als auch eukaryotische Zellen sein, Beispiele für prokaryotische Wirtszellen sind *E. coli* und für eukaryotische Zellen *Saccharomyces cerevisiae* oder Insektenzellen.

Ein besonders bevorzugte erfindungsgemäß verwendbare transformierte Wirtszelle ist eine transgene embryonale nichtmenschliche Stammzelle, die dadurch gekennzeichnet ist, daß sie ein erfindungsgemäß verwendbares knock-out Genkonstrukt oder eine erfindungsgemäß verwendbare Expressionskassette umfaßt. Verfahren zur Transformation von Wirtszellen und/oder Stammzellen sind dem Fachmann gut bekannt und umfassen zum Beispiel Elektroporation oder Mikroinjektion.

Eine weitere Ausführungsform der Erfindung ist die Verwendung eines transgenen nichtmenschlichen Säugetieres, dessen Genom ein vorangehend beschriebenes knock-out Genkonstrukt oder eine vorangehend beschriebene Expressionskassette zur Analyse und/oder Diagnose von Haut- und Darmerkrankungen und/oder von Wundheilung und/oder deren krankhaften Störungen, umfaßt. Transgene Tiere, die eine der vorangehend beschriebenen Expressionskassetten enthalten zeigen abhängig vom verwendeten Promotor beispielsweise im allgemeinen eine gewebespezifisch erhöhte Expression der Nukleinsäuren und/oder Polypeptide und lassen sich zur Analyse von Wundheilungsstörungen verwenden. So weist beispielsweise eine Activin A transgene Maus eine verbesserte Wundheilung auf (Munz et al., 1999, EMBO J. 18:5205-15) während eine transgene Maus mit dominant negativem KGF Rezeptor eine verzögerte Wundheilung aufweist (Werner et al., 1994, Science 266:819-22). Darüber hinaus können vorangehend beschriebene transgene Tiere mit beschleunigter Wundheilung ausgestattet werden.

Verfahren zur Herstellung von transgenen Tieren, insbesondere der Maus, sind dem Fachmann ebenfalls aus der DE 196 25 049 und den US 4,736,866; US 5,625,122; US 5,698,765; US 5,583,278 und US 5,750,825 bekannt und umfassen transgene Tiere, die beispielsweise über direkte Injektion von Expressionsvektoren (s.o.) in Embryonen oder Spermatozyten oder über die Transfektion von Expressionsvektoren in embryonale Stammzellen erzeugt werden können (Polites und Pinkert: DNA Microinjection and Transgenic Animal Produktion, Seite 15 bis 68 in Pinkert, 1994: Transgenic animal technology: a laboratory handbook, Academic Press, London, UK; Houdebine, 1997, Harwood Academic Publishers, Amsterdam, The Netherlands; Doetschman: Gene Transfer in Embryonic Stem Cells, Seite 115 bis 146 in Pinkert, 1994, supra; Wood: Retrovirus-Mediated Gene Transfer, Seite 147 bis 176 in Pinkert, 1994, supra; Monastersky: Gene Transfere Technology: Alternative Techniques and Applications, Seite 177 bis 220 in Pinkert, 1994, supra).

Werden erfindungsgemäß verwendete Nukleinsäuren in sogenannte Targeting Vektoren intergriert (Pinkert, 1994, supra) können nach Transfektion von embryonalen Stammzellen und homologer Rekombination beispielsweise knock-out Mäuse generiert werden, die im allgemeinen als heterozygote Mäuse verringerte Expression der Nukleinsäure zeigen, während homozygote Mäuse keine Expression der Nukleinsäure mehr aufweisen. Auch die so erzeugten Tiere lassen sich zur Analyse von Wundheilungsstörungen verwenden. So weisen beispielsweise die eNOS- (Lee et al., 1999, Am. J. Physiol. 277:H1600-H1608), Nf-1 (Atit et al., 1999, J. Invest. Dermatol. 112:835-42) und Osteopontin (Liaw et al., 1998, J. Clin. Invest. 101:967-71) knock-out Mäuse eine verschlechterte Wundheilung auf. Auch hier ist eine gewebespezifische Reduktion der Expression wundheilungsrelevanter Gene, beispielsweise in hautspezifischen Zellen unter Verwendung des Cre-loxP Systems (stat3 knock-out, Sano et al., EMBO J. 1999 18:4657-68), besonders zu bevorzugen. So erzeugte transgene und knock-out Zellen oder Tiere lassen sich auch zur Rasteruntersuchung (Screening) und zur Identifizierung von pharmakologisch aktiven Substanzen bzw. gentherapeutisch aktiven Vektoren verwenden.

Eine weitere Ausführungsform der Erfindung betrifft die Verwendung mindestens eines erfindungsgemäß verwendeten Polypeptids und/oder mindestens einer erfindungsgemäß verwendeten Nukleinsäure zur Analyse und/oder Diagnose und/oder Prävention und/oder Behandlung von Haut- und Darmerkrankungen und/oder von Wundheilung und/oder deren krankhaften Störungen, und/oder zur Identifizierung von pharmakologisch aktiven Substanzen in einer geeigneten Wirtszelle,.

Das Polypeptid wird beispielsweise durch Expression der vorangehend beschriebenen Nukleinsäure in einem geeigneten Expressionssystem, wie oben bereits beschrieben, nach dem Fachmann allgemein bekannten Methoden hergestellt. Als Wirtszellen eignen sich beispielsweise die *E. coli* Stämme DHS, HB101 oder BL21, der Hefestamm *Saccharomyces cerevisiae*, die Insektenzellinie Lepidopteran, z. B. von *Spodoptera frugiperda*, oder die tierischen Zellen COS, Vero, 293, HaCaT, und HeLa, die alle allgemein erhältlich sind.

Eine weitere Ausführungsform der Erfindung betrifft die Verwendung von Fusionsproteinen, die durch Expression von erfindungsgemäß verwendbarer Nukleinsäuren in einer geeigneten Wirtszelle hergestellt werden, zur Analyse und/oder Diagnose und/oder Prävention und/oder Behandlung von Haut- und Darmerkrankungen und/oder von Wundheilung und/oder deren krankhaften Störungen und/oder zur Identifizierung von pharmakologisch aktiven Substanzen in einer geeigneten Wirtszelle. Die Fusionsproteine selbst weisen bereits die Funktion eines vorangehend beschriebenen Polypeptids auf oder sind erst nach Abspaltung des Fusionsanteils die spezifische Funktion funktionell aktiv. Vor allem zählen hierzu Fusionsproteine mit einem Anteil von ca. 1-200, vorzugsweise ca. 1-150, insbesondere ca. 1-100, vor allem ca. 1-50 fremden Aminosäuren. Beispiele solcher Peptidsequenzen sind prokaryotische Peptidsequenzen, die z. B. aus der Galactosidase von E. coli abgeleitet sein können. Weiterhin können auch virale Peptidsequenzen, wie zum Beispiel vom Bakteriophagen M13 verwendet werden, um so Fusionsproteine für das dem Fachmann bekannte "phage display"Verfahren zu erzeugen.

Weitere bevorzugte Beispiele für Peptidsequenzen für Fusionsproteine sind Peptide, die die Detektion des Fusionsproteins erleichtern, hierzu zählen beispielsweise "Green-fluorescent-protein" (WO 95/07463) oder funktionelle Varianten davon.

Zur Aufreinigung der vorangehend beschriebenen Proteine kann ein weiteres/weiterer Polypeptid("tag") angefügt sein. Geignete Protein-tags erlauben beispielsweise die hochaffine Absorption an eine Matrix, stringentes Waschen mit geeigneten Puffern, ohne den Komplex in nennenswertem Maße zu eluieren und anschließend gezielte Elution des absorbierten Komplexes. Beispiele der dem Fachmann bekannten Protein-tags sind ein (His)₆-tag, ein Myc-tag, ein FLAG-tag, ein Strep-tag, ein Strep-tag II, ein Hämagglutinin-tag, Glutathion-Transferase (GST)-tag, Intein mit einem Affinitäts-Chintin-binding-tag oder Maltose-binding protein (MBP)-tag. Diese Protein-tags können sich N-, C-terminal und/oder intern befinden.

Ein weitere Ausführungsform der Erfindung betrifft die Verwendung eines Antikörpers, vorzugsweise eines polyklonalen oder monoklonalen Antikörpers zur Analyse und/oder Diagnose und/oder Prävention und/oder Behandlung von Haut- und Darmerkrankungen und/oder von Wundheilung und/oder deren krankhaften Störungen und/oder zur Identifizierung von pharmakologisch aktiven Substanzen. So kann beispielsweise die lokale Injektion von monoklonalen Antikörpern gegen TGF beta 1 im Tiermodell die Wundheilung verbessern (Ernst et al., 1996, Gut 39:172-5).

Zur Herstellung des Antikörpers wird ein Polypeptid oder funktionelle Varianten davon oder Teile davon mit mindestens 6 Aminosäuren, vorzugsweise mit mindestens 8 Aminosäuren, insbesondere mit mindestens 12 Aminosäuren gemäß der vorliegenden Erfindung verwendet. Das Verfahren erfolgt nach dem Fachmann allgemein bekannten Methoden durch Immunisieren eines Säugetiers, beispielsweise eines Kaninchens, mit dem vorangehend beschriebenen Polypeptid oder den genannten Teilen davon, gegebenenfalls in Anwesenheit von z. B. Freund's Adjuvant und/oder Aluminiumhydroxidgelen (siehe z. B. Diamond, B. A. et al. (1981) The New England Journal of Medicine, 1344-1349). Die im Tier aufgrund einer immunologischen Reaktion entstandenen polyklonalen Antikörper lassen sich anschließend nach allgemein bekannten Methoden leicht aus dem Blut isolieren und z. B. über Säulenchromatographie reinigen. Monoklonale Antikörper können beispielsweise nach der bekannten Methode von Winter & Milstein (Winter, G. & Milstein, C. (1991) Nature, 349, 293-299) hergestellt werden.
Als Alternative zu den klassischen Antikörpern können beispielsweise auf Lipocalin basierende sogenannte "Anticaline" verwendet werden (Beste et al., 1999, Proc. Natl. Acad. Sci. USA, 96:1898-1903). Die natürlichen Liganden-bindenden Sites der Lipocaline, wie z.B. das Retinol-bindende Protein oder das Bilinbindende Protein können beispielsweise durch einen "kombinatorischen Protein-Design" Ansatz in einer Weise verändert werden, daß sie an ausgewählte Haptene, beispielsweise an die erfindungsgemäß verwendbaren Polypeptide binden (Skerra, 2000, Biochim. Biophys. Acta 1482:337-50). Weitere bekannte "scaffolds" als Alternativen für Antikörper für die molekulare Erkennung sind bekannt (Skerra, J. Mol. Recognit., 2000, 13:167-187).

Der erfindungsgemäß verwendete Antikörper, der gegen ein vorangehend beschriebenes Polypeptid gerichtet ist und mit den vorangehend beschriebenen Polypeptiden spezifisch reagiert, wobei die oben genannten Teile des Polypeptids entweder selbst immunogen sind oder durch Kopplung an geeignete Träger, wie z. B. bovines Serumalbumin, immunogen gemacht bzw. in ihrer Immunogenität gesteigert werden können, ist entweder polyklonal oder monoklonal, bevorzugt ist ein monoklonaler Antikörper. Unter dem Begriff Antikörper versteht man gemäß der vorliegenden Erfindung auch gentechnisch hergestellte und gegebenenfalls modifizierte Antikörper bzw. antigenbindende Teile davon, wie z.B. chimäre Antikörper, humanisierte Antikörper, multifunktionelle Antikörper, bi- oder oligospezifische Antikörper, einzelsträngige Antikörper, F(ab)- oder F(ab)₂-Fragmente (siehe z.B. EP-B1-0 368 684, US 4,816,567, US 4,816,397, WO 88/01649, WO 93/06213, WO 98/24884).

Die vorliegende Erfindung betrifft weiterhin die Verwendung mindestens einer erfindungsgemäß verwendbare Nukleinsäure, mindestens eines erfindungsgemäß verwendbaren Polypeptids oder mindestens eines Antikörper gemäß der vorliegenden Erfindung, gegebenenfalls zusammen mit geeigneten Zusatz- und/oder Hilfsstoffen, zur Herstellung eines Arzneimittels zur Behandlung und/oder Prävention von Haut- und/oder Darmerkrankungen und/oder von Wundheilung und/oder deren krankhaften Störungen.

Die Therapie von Haut- und/oder Darmerkrankungen und/oder von Wundheilung und/oder deren krankhaften Störungen kann auf herkömmliche Weise, z.B. durch Verbände, Pflaster, Kompressen oder Gele erfolgen, die die erfindungsgemäßen Arzneimittel enthalten. So ist es möglich, die geeigneten Zusatz- oder Hilfsstoffe, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., enthaltenden Arzneimittel topisch und lokal zu verabreichen, um die Wundheilung sofort und unmittelbar zu beeinflussen. Die Verabreichung der erfindungsgemäßen Arzneimittel kann weiterhin gegebenenfalls in Form von Liposomenkomplexen bzw. Goldpartikelkomplexen ebenfalls topisch und lokal im Bereich der Wunde erfolgen. Weiterhin kann die Behandlung mittels eines transdermalen therapeutischen Systems (TTS) erfolgen, das eine zeitlich gesteuerte Abgabe der erfindungsgemäß verwendbaren Arzneimittel ermöglicht. Die Behandlung mittels der erfindungsgemäß verwendbaren Arzneimittel kann aber auch über orale Dosierungsformen, wie z.B. Tabletten oder Kapseln, über die Schleimhäute, zum Beispiel der Nase oder der Mundhöhle, oder in Form von unter die Haut implantierten Dispositorien erfolgen. TTS sind zum Beispiel aus den EP 0 944 398 A1, EP 0 916 336 A1, EP 0 889 723 A1 oder EP 0 852 493 A1 bekannt.

Für die gentherapeutische Anwendung beim Menschen ist vor allem ein Arzneimittel geeignet, das die erfindungsgemäß verwendete Nukleinsäure in nackter Form oder in Form eines der oben beschriebenen gentherapeutisch wirksamen Vektoren oder in mit Liposomen bzw. Goldpartikeln komplexierter Form enthält. Der pharmazeutische Träger ist beispielsweise eine physiologische Pufferlösung, vorzugsweise mit einem pH von ca. 6,0-8,0, vorzugsweise von ca. 6,8-7,8. Insbesondere von ca. 7,4 und/oder einer Osmolarität von ca. 200-400 milliosmol/Liter, vorzugsweise von ca. 290-310 milliosmol/Liter. Zusätzlich kann der pharmazeutische Träger geeignete Stabilisatoren, wie z. B. Nukleaseinhibitoren, vorzugsweise Komplexbildner wie EDTA und/oder andere dem Fachmann bekannte Hilfsstoffe enthalten.

Die Verabreichung der vorangehend beschriebenen Nukleinsäure gegebenenfalls in Form der oben näher beschriebenen Virusvektoren oder als Liposomenkomplexe bzw. Goldpartikelkomplexe erfolgt üblicherweise topisch und/oder lokal im Bereich der Wunde. Es ist auch möglich, das Polypeptid selbst mit geeigneten Zusatz- oder Hilfsstoffen, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., zu verabreichen, um die Wundheilung sofort und unmittelbar zu beeinflussen.

Ein weiterer Gegenstand der vorliegenden Erfindung betrifft die Verwendung eines Diagnostikums zur Diagnose von Haut- und/oder Darmerkrankungen oder Erkrankungen bei der Wundheilung, das mindestens eine Nukleinsäure, mindestens ein Polypeptid oder mindestens einen Antikörper gemäß der Erfindung, gegebenenfalls zusammen mit geeigneten Zusatz- und/oder Hilfsstoffen, umfaßt.

Beispielsweise kann erfindungsgemäß verwendbares Diagnostikum mit Hilfe einer erfindungsgemäß verwendeten Nukleinsäure auf der Basis der Polymerasekettenreaktion (Beispiel 2, PCR-Diagnostik, z. B. gemäß EP 0 200 362) oder eines RNase-Protection-Assays, wie in Beispiel 3 näher dargestellt, hergestellt werden. Diese Tests beruhen auf der spezifischen Hybridisierung der erfindungsgemäß verwendeten Nukleinsäuren mit dem komplementären Gegenstrang, üblicherweise der entsprechenden mRNA. Die erfindungsgemäß verwendete Nukleinsäure kann hierbei auch modifiziert sein, wie z. B. in EP 0 063 879 beschrieben. Vorzugsweise wird ein erfindungsgemäß verwendetes DNA-Fragment mittels geeigneter Reagenzien, z. B. radioaktiv mit α-P³²-dCTP oder nicht-radioaktiv mit Biotin oder Digoxigenin, nach allgemein bekannten Methoden markiert und mit isolierter RNA, die vorzugsweise vorher an geeignete Membranen aus z. B. Nitro-Cellulose oder Nylon gebunden wurde, inkubiert. Bei gleicher Menge an untersuchter RNA aus jeder Gewebeprobe kann somit die Menge an mRNA bestimmt werden, die spezifisch durch die Sonde markiert wurde und mit der Menge an mRNA aus gesundem Gewebe verglichen werden. Alternativ kann die Bestimmung an mRNA auch in Gewebeschnitten mit Hilfe der in situ Hybridisierung (siehe Beispiele 4, 7 und Werner et al., 1992, Proc. Natl. Acad. Sci. U S A 89:6896-6900) erfolgen.

Mit Hilfe des erfindungsgemäß verwendbaren Diagnostikums kann somit auch eine Gewebeprobe *in vitro* auf die Expressionsstärke des korrespondierenden Gens spezifisch gemessen werden, um eine mögliche Wundheilungsstörung, Darmerkrankung oder dermatologische Erkrankungen sicher diagnostizieren zu können (Beispiele 1, 2, 3, 5, 6). Insbesondere eignet sich ein solches Verfahren zum Beispiel zur frühzeitigen Prognose von Störungen.

Ein weiteres erfindungsgemäß verwendbares Diagnostikum enthält das erfindungsgemäß verwendbare Polypeptid bzw. die oben näher beschriebenen immunogenen Teile davon. Das Polypeptid bzw. die Teile davon, die vorzugsweise an eine Festphase, z. B. aus Nitrocellulose oder Nylon, gebunden sind, können beispielsweise mit der zu untersuchenden Körperflüssigkeit, z. B. Wundsekret, *in vitro* in Berührung gebracht werden, um so beispielsweise mit Autoimmunantikörper reagieren zu können. Der Antikörper-Peptid-Komplex kann anschließend beispielsweise anhand markierter Antihuman-IgG- oder Antihuman-IgM-Antikörper nachgewiesen werden. Bei der Markierung handelt es sich beispielsweise um ein Enzym, wie Peroxidase, das eine Farbreaktion katalysiert. Die Anwesenheit und die Menge an anwesenden Autoimmunantikörper kann somit über die Farbreaktion leicht und schnell nachgewiesen werden.

Ein anderes erfindungsgemäß verwendbares Diagnostikum enthält die erfindungsgemäß verwendbaren Antikörper selbst. Mit Hilfe dieser Antikörper kann beispielsweise eine Gewebeprobe leicht und schnell dahingehend untersucht werden, ob das betreffende Polypeptid in einer erhöhten Menge vorhanden ist, um dadurch einen Hinweis auf eine mögliche Wundheilungsstörung zu erhalten. In diesem Fall sind die erfindungsgemäß verwendbaren Antikörper beispielsweise mit einem Enzym, wie oben bereits beschrieben, markiert. Der spezifische Antikörper-Peptid-Komplex kann dadurch leicht und ebenso schnell über eine enzymatische Farbreaktion nachgewiesen werden.

Ein weiteres erfindungsgemäß verwendbares Diagnostikum umfaßt eine Sonde, vorzugsweise eine DNA-Sonde, und/oder Primer. Dies eröffnet eine weitere Möglichkeit, die erfindungsgemäß verwendbaren Nukleinsäuren, zum Beispiel durch die Isolierung aus einer geeigneten Genbank, beispielsweise aus einer wundspezifischen Genbank, anhand einer geeigneten Sonde zu erhalten (siehe z. B. J. Sambrook et al., 1989, Molecular Cloning. A Laboratory Manual, 2. Ausgabe, Cold Spring Harbor Laboratory, Cold Spring Harbor, NY Kapitel 8 Seite 8.1 bis 8.81, Kapitel 9 Seite 9.47 bis 9.58 und Kapitel 10 Seite 10.1 bis 10.67).

Als Sonde eignen sich beispielsweise DNA- oder RNA-Fragmente mit einer Länge von ca. 100-1000 Nukleotiden, vorzugsweise mit einer Länge von ca. 200-500 Nukleotiden, insbesondere mit einer Länge von ca. 300-400 Nukleotiden deren Sequenz aus den Polypeptiden gemäß den SEQ ID Nr. 1 bis SEQ ID Nr. 10 des Sequenzprotokolls und/oder anhand der cDNA Sequenzen der in den Figur 5 angegebenen Datenbankeinträge abgeleitet werden kann (siehe auch Beispiele 3, 5, 6).

Alternativ können anhand der abgeleiteten Nukleinsäuresequenzen Oligonukleotide synthetisiert werden, die sich als Primer für eine Polymerase Kettenreaktion eignen. Mit diesen kann die erfindungsgemäß verwendbare Nukleinsäure oder Teile dieser aus cDNA, beispielsweise wundspezifischer cDNA, amplifiziert und isoliert werden (Beispiele 2, 5, 6). Als Primer eignen sich beispielsweise DNA-Fragmente mit einer Länge von ca. 10-100 Nukleotiden, vorzugsweise mit einer Länge von ca. 15 bis 50 Nukleotiden, insbesondere mit einer Länge von 20-30 Nukleotiden deren Sequenz aus den Polypeptiden gemäß den SEQ ID Nr. 1 bis SEQ ID Nr. 10 des Sequenzprotokolls und/oder anhand der cDNA Sequenzen der in der Figur 5 angegebenen Datenbankeinträge abgeleitet werden kann (Beispiele 2, 5, 6).

Die Erfindung umfaßt weiterhin einen erfindungsgemäß verwendbaren Test zur Identifizierung funktioneller Interaktoren in Zusammenhang mit Haut- und/oder Darmerkrankungen und/oder mit der Wundheilung und/oder mit deren krankhaften Störungen, der mindestens eine Nukleinsäure, mindestens ein Polypeptid oder mindestens einen Antikörper gemäß der vorliegenden Erfindung, gegebenenfalls zusammen mit geeigneten Zusatz- und/oder Hilfsstoffen, umfaßt.

Unter dem Begriff "funktionelle Interaktoren" im Sinne der vorliegenden Erfindung sind alle diejenigen Moleküle, Verbindungen und/oder Zusammensetzungen und Stoffgemische zu verstehen, die mit den vorangehend beschriebenen Nukleinsäuren, Polypeptiden oder Antikörpern, gegebenenfalls zusammen mit geeigneten Zusatz- und/oder Hilfsstoffen, unter geeigneten Bedingungen in Wechselwirkung treten können. Mögliche Interaktoren sind einfache chemische organische oder anorganische Moleküle oder Verbindungen, können aber auch Nukleinsäuren, Peptide, Proteine oder Komplexe davon umfassen. Die funktionellen Interaktoren können aufgrund ihrer Wechselwirkung die Funktion(en) der Nukleinsäuren, Polypeptide oder Antikörper *in vivo* oder *in vitro* beeinflussen oder auch nur an die vorangehend beschriebenen Nukleinsäuren, Polypeptide oder Antikörper binden oder mit ihnen andere Wechselwirkungen kovalenter oder nicht-kovalenter Weise eingehen.

Ein geeignetes erfindungsgemäß verwendbares Testsystem läßt sich beispielsweise durch die stabile Transfektion von epidermalen bzw. dermalen Zellen mit Expressionsvektoren, die selektierbare Markergene und die erfindungsgemäß verwendeten Nukleinsäuren enthalten, herstellen. Bei diesem Verfahren wird die Expression der erfindungsgemäß verwendeten Nukleinsäuren in den Zellen so verändert, daß sie der pathologisch gestörten Expression *in vivo* entspricht. Auch antisense Oligonukleotide, die die erfindungsgemäß verwendbaren Nukleinsäuresequenzen enthalten, können zu diesem Zweck eingesetzt werden. Von besonderem Vorteil für diese Systeme ist es daher, das Expressionsverhalten der Gene bei gestörten regenerativen Prozessen, wie in dieser Anmeldung offengelegt, zu kennen. Oft kann so das pathologische Verhalten der Zellen *in vitro* nachgeahmt werden und es können Substanzen gesucht werden, die das normale Verhalten der Zellen wieder herstellen und die ein therapeutisches Potential besitzen.

Für diese erfindungsgemäß verwendbaren Testsysteme eignen sich z.B. HaCaT-Zellen, die allgemein erhältlich sind, und der Expressionsvektor pCMV4 (Anderson et al., 1989, J. Biol. Chem. 264:8222-9). Die erfindungsgemäß verwendbare Nukleinsäure kann dabei sowohl in sense als auch in antisense Orientierung in die Expressionsvektoren integriert werden, so daß die funktionelle Konzentration an mRNA der entsprechenden Gene in den Zellen entweder erhöht, oder durch Hybridisierung mit der antisense-RNA erniedrigt wird. Nach der Transfektion und Selektion stabiler Transformanden zeigen die Zellen in Kultur im allgemeinen ein verändertes Proliferations-, Migrations, und/oder Differenzierungsverhalten im Vergleich zu Kontrollzellen. Dieses Verhalten *in vitro* ist häufig mit der Funktion der entsprechenden Gene bei regenerativen Prozessen im Organismus korreliert (Yu et al., 1997, Arch. Dermatol. Res. 289:352-9; Mils er al., 1997, Oncogene 14: 15555-61; Charvat et al., 1998, Exp Dermatol 7: 184-90; Mythily et al., 1999, J. Gen. Virol. 80:1707-13; Werner, 1998, Cytokine Growth Factor Rev. 9:153-65) und läßt sich mit einfachen und schnell durchzuführenden Tests nachweisen, so daß man darauf basierend Testsysteme für pharmakologisch aktive Substanzen aufbauen kann. So läßt sich das Proliferationsverhalten von Zellen sehr schnell durch z.B. den Einbau von markierten Nukleotiden in die DNA der Zellen (siehe z.B. de Fries und Mitsuhashi, 1995, J. Clin. Lab. Anal. 9:89-95; Perros und Weightman, 1991, Cell Prolif. 24:517-23; Savino und Dardenne, 1985, J. Immunol. Methods 85:221-6), durch Anfärbung der Zellen mit spezifischen Farbstoffen (Schulz et al., 1994, J. Immunol. Methods 167:1-13) oder über immunologische Verfahren (Frahm et al., 1998, J. Immunol. Methods 211:43-50) nachweisen. Die Migration läßt sich einfach durch den "Migration Index" Test (Charvat et al., supra) und vergleichbare Testsysteme (Benestad et al., 1987, Cell Tissue Kinet. 20:109-19, Junger et al., 1993, J. Immunol. Methods 160:73-9) nachweisen. Als Differenzierungsmarker eignen sich z.B. Keratin 6, 10 und 14 sowie Loricrin und Involucrin (Rosenthal et al., 1992, J, Invest, Dermatol, 98:343-50), deren Expression z.B. über allgemein erhältliche Antikörper leicht nachzuweisen ist.

Ein anderes geeignetes Testsystem basiert auf der Identifikation funktioneller Interaktionen mit dem sogenannten "Two-Hybrid System" (Fields und Sternglanz, 1994, Trends in Genetics, 10, 286-292; Colas und Brent, 1998 TIBTECH, 16, 355-363). Bei diesem Test werden Zellen mit Expressionsvektoren transformiert, die Fusionsproteine aus dem erfindungsgemäß verwendbaren Polypeptid und einer DNA-Bindungsdomäne eines Transkriptionsfaktors wie beispielsweise Gal4 oder LexA exprimieren. Die transformierten Zellen enthalten außerdem ein Reportergen, dessen Promotor Bindungsstellen für die entsprechende DNA-Bindungsdomäne enthalten. Durch Transformation eines weiteren Expressionsvektors, der ein zweites Fusionsprotein aus einem bekannten bzw. unbekannten Polypeptid mit einer Aktivierungsdomäne, beispielsweise von Gal4 oder Herpes Virus VP16, exprimiert, kann die Expression des Reportergens stark gesteigert werden, wenn das zweite Fusionsprotein mit dem erfindungsgemäß verwendbaren Polypeptid funktionell interagiert. Diese Expressionssteigerung kann man ausnutzen, um neue Interaktoren zu identifizieren, beispielsweise indem man zur Konstruktion des zweiten Fusionsproteins eine cDNA-Bibliothek aus sich regenerierendem Gewebe herstellt. Zudem läßt sich dieses Testsystem zur Rasteruntersuchung von Substanzen ausnutzen, die eine Interaktion zwischen dem erfindungsgemäß verwendbaren Polypeptid und einem funktionellen Interaktor inhibieren.

Solche Substanzen verringern die Expression des Reportergens in Zellen, die Fusionsproteine des erfindungsgemäß verwendbaren Polypeptids und des Interaktors exprimieren (Vidal und Endoh, 1999, Trends in Biotechnology, 17:374-81). So lassen sich schnell neue Wirkstoffe identifizieren, die zur Therapie von Störungen regenerativer Prozesse eingesetzt werden können.

Funktionelle Interaktoren der erfindungsgemäß verwendbaren Polypeptide können auch Nukleinsäuren sein, die über Selektionsverfahren, wie beispielsweise SELEX (siehe Jayasena, 1999, Clin. Chem. 45:1628-50; Klug und Famulok, 1994, M. Mol. Biol. Rep. 20:97-107; Toole et al., 1996, US 5582981) isoliert wereden. Im SELEX-Verfahren werden typischerweise aus einem großen Pool unterschiedlicher, einzelsträngige RNA Moleküle durch wiederholte Amplifikation und Selektion diejenigen Moleküle isoliert, die an Polypeptide mit hoher Affinität binden (Aptamere). Aptamere können auch in ihrer spiegelbildlichen Form, beispielsweise als L-Ribonukleotid, synthetisiert und selektioniert werden (Holte et al., 1996, Nat. Biotechnol. 14:1116-9; Klussmann et al., 1996, Nat. Biotechnol. 14:1112-5). So isolierte Formen haben den Vorteil, daß sie nicht von natürlich vorkommenden Ribonukleasen abgebaut werden und daher größere Stabilität besitzen.

Ein weiterer Gegenstand der Erfindung ist die Verwendung eines auf einem Trägermaterial fixierten Arrays zur Analyse in Zusammenhang mit Haut- und/oder Darmerkrankungen oder mit der Wundheilung und/oder mit deren krankhaften Störungen, wobei das Array mindestens eine Nukleinsäure und/oder mindestens ein Polypeptid und/oder oder mindestens einen Antikörper gemäß der vorliegenden Erfindung umfaßt.

Verfahren zur Herstellung von solchen Arrays sind zum Beispiel aus WO 89/109077, WO 90/15070, WO 95/35505 und US 5,744,305 mittels Spottings, Druckens oder Festphasenchemie in Verbindung mit photolabilen Schutzgruppen bekannt.

Ein weiterer Gegenstand der Erfindung umfaßt die Verwendung eines DNA-Chip und/oder Protein-Chip zur Analyse in Zusammenhang mit Haut- und/oder Darmerkrankungen und/oder mit der Wundheilung und/oder mit deren krankhaften Störungen, der mindestens eine Nukleinsäure und/oder mindestens ein Polypeptid und/oder oder mindestens einen Antikörper gemäß der vorliegenden Erfindung umfaßt. DNA-Chips sind zum Beispiel aus der US 5,837,832 bekannt.

Die Erfindung soll nun im folgenden anhand der Figuren und Beispiele weiter verdeutlicht werden, ohne daß die Erfindung hierauf eingeschränkt wird.

### Beschreibung der Tabellen, Figuren und Sequenzen:

- Figur 1:: Autoradiogramme von Hybridisierungen von Membranen mit gleichem Muster von aufgebrachten cDNA-Fragmenten mit vier verschiedenen Sonden. Die cDNA Fragmente entstammten alle einer wundspezifischen, subtraktiven cDNA-Bibliothek, die für solche cDNAs angereichert war, die in Wundgewebe stärker im Vergleich zur intakten Haut exprimiert wurden. Alle Sonden wurden aus cDNAs hergestellt, die aus subtraktiven Hybridisierungen stammten. A: wundspezifische Sonde (Subtraktion Wunde versus intakte Haut), B: hautspezifische Sonde (Subtraktion intakte Haut versus Wunde), C: Sonde spezifisch für schlecht heilende Wunden (Subtraktion Wunde Dexamethason behandelte Tiere versus Wunde Kontrolltiere), D: Sonde spezifisch für gut heilende Wunden (Subtraktion Wunde Kontrolltiere versus Wunde Dexamethason behandelte Tiere). Die Positionen der NM23-M2 cDNA (jeweils doppelt aufgetragen) sind mit Pfeilen gekennzeichnet.
- Figur 2:: Ergebnisse der quantitativen "real time RTPCR" von NM23-M2 mit verschiedenen Wundheilungsstadien der Maus. Die Formel zur Berechnung der Abundanz relativ zu GAPDH ist angegeben. Die Induktionen ergeben sich durch die Normalisierung der Abundanz auf die Abundanz in der intakten Haut.
- Figur 3:: Ergebnisse des RNase Protection Assays von NM23-H1 mit Hautproben von Psoriasis-Patienten und Kontrollpersonen. Die radioaktive Hybridisierungssonde ohne RNase-Behandlung (Spuren 1 und 5) sowie die Negativkontrolle (tRNA, Spuren 2 und 6), die RNA aus Hautbiopsien von 4 verschiedenen Kontrollpatienten (Spuren 3, 7, 8 und 9) und die RNA aus Hautbiopsien von 3 verschiedenen Psoriasispatienten (Spuren 4, 10 und 11), jeweils nach Hybridisierung mit der Sonde und RNase-Behandlung, wurden aufgetragen. Die Pfeile zeigen die Position des nach Hybridisierung mit der NM23-H1 mRNA vor RNase-Abbau geschützten RNA-Fragments der Sonde.
- Figur 4:: Ergebnisse des RNase Protection Assays von NM23-H1 mit Darmproben von Morbus Crohn Patienten und Kontrollpersonen. Die radioaktive Hybridisierungssonde ohne RNAse-Behandlung (Spur 1) sowie die Negativkontrolle (tRNA, Spur 2), die RNA aus Darmbiopsien eines Kontrollpatienten (Spur 3), die RNA aus Darmbiopsien von Morbus Crohn Patienten mit wenig entzündlichen Arealen (Spuren 4 und 6) und deutlich entzündlichen Arealen (Spuren 5, 7 und 8), jeweils nach Hybridisierung mit der Sonde und RNase-Behandlung, wurden aufgetragen. Die eingesetzte RNA in den Spuren 4, 5 und 6 sowie in den Spuren 7 und 8 entstammen jeweils aus dem selben Patienten. Der Pfeil zeigt die Position des nach Hybridisierung mit der NM23-H1 mRNA vor RNase-Abbau geschützte RNA-Fragments der Sonde.
- Figur 5:: Tabellarische Übersicht über die bei der Analyse der Genexpression während des Wundheilungsprozesses identifizierten Polypeptidsequenzen der NM23 Genfamilie und ihre cDNAs und Zugangsnummern.
- Figur 6:: Vergleich der Polypeptidsequenz der identifizierten Proteine von NM23A (NDKA_human, NDKA_maus) und NM23B (NDKB_human, NDKB_maus) aus Maus und Mensch. Abweichungen zur humanen Sequenz von NM23A sind markiert.
- Figur 7:: Tabellarische Übersicht über die mittels "TaqMan Assay" bestimmte Menge an cDNA von NM23-M1 und NM23-M2 zu verschiedenen Zeitpunkten nach Verletzung der adulten Maus.
- Figur 8:: Tabellarische Übersicht über die mittels "TaqMan Assay" bestimmte Menge an mRNA wundrelevanter Gene in humanen Tag 1- und Tag 5-Wunden.
SEQ ID Nr. 1 bis SEQ ID Nr. 10 zeigen die erfindungsgemäß verwendeten Polypeptid-Sequenzen aus Mensch oder Maus.
SEQ ID Nr. 11 bis SEQ ID Nr. 14 und SEQ ID Nr. 17 bis SEQ ID Nr. 26 zeigen DNA-Sequenzen von Oligonukleotiden, die für die Versuche der vorliegenden Erfindung verwendet wurden.
SEQ ID Nr. 15 bis SEQ ID Nr.16 zeigen DNA-Sequenzen von NM23, die zur Herstellung von Sonden für RNAse Protection Assay und in situ Hybridisierung verwendet wurden.

### Beispiele

### Beispiel 1: Anreicherung von wundrelevanter cDNA mittels subtraktiver Hybridisierung und Identifizierung von NM23-M2 als wundrelevantes Gen

Aus intakter Haut und aus Wundgewebe (Verwundung am Rücken 1 Tag vor Gewebeentnahme durch Scherenschnitt) von BALB/c-Mäusen wurde durch Standardmethoden (Chomczynski und Sacchi, 1987, Anal. Biochem. 162: 156-159, Chomczynski und Mackey, 1995, Anal. Biochem. 225: 163-164) Gesamt-RNA isoliert. Um Gewebe von Mäusen mit schlecht heilenden Wunden zu gewinnen, wurden BALB/c-Mäuse vor der Verwundung mit Dexamethason (Injektion von 0,5 mg Dexamethason in isotonischer Salzlösung pro kg Körpergewicht zwei mal pro Tag für 5 Tage) behandelt. Die RNAs wurden dann mit Hilfe einer reversen Transkriptase in cDNA umgeschrieben. Die cDNA-Synthese erfolgte mit dem "SMART PCR cDNA Synthesis Kit" der Firma Clontech Laboratories GmbH, Heidelberg, nach Anweisungen des entsprechenden Manuals.

Um diejenigen cDNAs zu identifizieren, die mit unterschiedlicher Häufigkeit in den cDNA-Pools vorkamen, wurde eine subtraktive Hybridisierung (Diatchenko et al., 1996, Proc. Natl. Acad. Sci. USA 93: 6025-30) durchgeführt. Dies erfolgte mit dem "PCR-Select cDNA Subtraction Kit" der Firma Clontech Laboratories GmbH, Heidelberg, nach Anweisungen des entsprechenden Manuals, wobei die Abtrennung überschüssiger Oligonukleotide nach der cDNA-Synthese über Agarose-Gelelekrophorese erfolgte. Es wurden vier für wundrelevante Gene angereicherte cDNA-Pools angelegt, wobei ein Pool für cDNA Fragmente angereichert war, die im Wundgewebe im Vergleich zu intakter Haut stärker exprimiert sind ("wundspezifischer cDNA-Pool"), ein Pool war angereichert an cDNA-Fragmenten, die in intakter Haut im Vergleich zu Wundgewebe stärker exprimiert sind ("hautspezifischer cDNA Pool"), ein Pool war angereichert an cDNA-Fragmenten, die in gut heilenden Wunde im Vergleich zu schlecht heilenden Wunden stärker exprimiert sind ("gut heilender cDNA-Pool") und ein Pool war angereichert an cDNA-Fragmenten, die in schlecht heilenden Wunden im Vergleich zu gut heilenden Wunden stärker exprimiert sind ("schlecht heilender cDNA-Pool").

Um diejenigen Gene zu identifizieren, die in den wundheilungsrelevanten cDNA-Pools enthalten waren, wurde das Vorhandensein der entsprechenden cDNAs in den Pools im "Reverse Northern Blot" analysiert. Hier werden cDNA-Fragmente auf Membranen in Form von Arrays von vielen verschiedenen cDNAs fixiert, und mit einem komplexen Gemisch radioaktiv markierter cDNA hybridisiert (Sambrook et al., 1989, Molecular cloning: A Laboratory Manual, Cold Spring Harbor, Cold Spring Harbor Laboratory Press, New York, Kapitel 9 Seite 9.47 bis 9.58 und Kapitel 10 Seite 10.38 bis 10.50; Anderson and Young: Quantitative filter hybridisation; in: Nucleic Acids Hybridisation, A Practical Approach, 1985, Eds. Hames and Higgins, IRL Press Ltd.; Oxford, Kapitel 4, Seite 73 bis 112).

Zur Herstellung geeigneter Hybridisierungssonden wurden die subtrahierten cDNA-Pools mit der Restriktionsendonuklease RsaI behandelt und über Agarosegelelektrophorese gereinigt (Sambrook et al., supra, Kapitel 6, Seite 6.1 bis 6.35), um die cDNA-Synthese- und Amplifikationsprimer (siehe Manual "PCR-Select cDNA Subtraction Kit", Clonetech) abzutrennen. Die cDNAs wurde dann mit der "random-hexamer priming" Methode (Feinberg und Vogelstein, 1983, Anal. Biochem. 132:6-13) radioaktiv markiert, um Hybridisierungssonden herzustellen.

Die Membran wurde für 30 min bei 65°C in 25 ml Hybridisierungslösung vorinkubiert (25 mM Natriumphosphat, pH = 7,5, 125 mM NaCl, 7% SDS). Die Hybridisierungssonde wurde 10 min bei 100°C denaturiert, anschließend auf Eis abgekühlt, ca. 100 CPM pro ml zur Hybridisierungslösung gegeben und die Hybridisierung für 16 Stunden bei 65°C im Hybridisierungsofen durchgeführt. Danach wurde die Membran zweimal für 10 min mit der Hybridisierungslösung ohne Sonde bei 65°C gewaschen. Anschließend wurde die Membran mehrfach für jeweils 10 min in Waschlösung (2,5 mM Natriumphosphat, pH = 7,5, 12,5 mM NaCl, 0,7% SDS) bei 65°C gewaschen, bis in der abgegossenen Lösung keine Aktivität mehr nachgewiesen werden konnte. Die radioaktiven Signale wurden mit einem Phosphoimager (BioRad, Quantitiy One®) ausgewertet (Figur 1). Danach wurden diejenigen cDNAs ausgewählt, die mit den verschiedenen Sonden unterschiedliche Signalintensitäten ergaben. Dabei ergab sich an der Position von NM23-M2 auf der Membran eine leicht stärkere Signalintensität mit der Hybridisierungssonde des wundspezifischen cDNA-Pools im Vergleich zum hautspezifischen cDNA-Pool und eine deutliche stärkere Signalintensität mit der Hybridisierungssonde des schlecht heilenden cDNA-Pools im Vergleich zum gut heilenden cDNA-Pool.

### Beispiel 2: Bestätigung des Expressionsmusters von NM23-M2 mittels "real time quantitative RT-PCR"

Eine Bestätigung der differentiellen Expression der erfindungsgemäß verwendeten Nukleinsäuren erfolgte über eine real-time RT-PCR im ABI Prism 7700 Sequence Detection System (PE Applied Biosystems). Das Gerät war mit der ABI Prism 7200/7700 SDS-Software Version 1.6.3 (1998) ausgestattet. Der Nachweis von PCR Produkten erfolgte während der Amplifikation der cDNA mit Hilfe des Farbstoffs SYBR Green 1, dessen Fluoreszenz durch Bindung an doppelsträngige DNA stark erhöht wird (Karlsen et al. 1995, J. Virol. Methods. 55: 153-6; Wittwer et al., 1997, BioTechniques 22:130-8, Morrison et al., 1998, BioTechniques 24: 954-62). Basis für die Quantifizierung ist der PCR-Zyklus ("threshold cycle", C_{T}-Wert), der erreicht ist, wenn das Fluoreszenzsignal einen definierten Schwellenwert übersteigt. Die Auswertung erfolgt über die △-C_{T}-Methode (User Bulletin #2, Relative Quantitation of Gene Expression, PE Applied Biosystems, 1997). Die Abundanzen der cDNAs wurden relativ zu einer endogenen Referenz (GAPDH) bestimmt. Die Ergebnisse sind in Figur 2 dargestellt.

Gesamt-RNA-Pools wurde wie oben beschrieben aus Haut und Wundgewebe von jeweils 16 Tieren gewonnen und 1 µg Gesamt-RNA wurde mit dem TaqMan Reverse Transcription Reagents Kit (PE) nach den Empfehlungen des Herstellers (SYBR Green® PCR and RT-PCR Reagents-Protokol, PE Applied Biosystems, 1998) in einem Thermocycler (GeneAmp PCR-System 9700, PE) revers transkribiert. Die Primer für die Amplifikation der NM23-M2 cDNA (*NM23*-Primer 1: TTCAAAACCAGGCACCATCC (SEQ ID Nr. 11), *NM23*-Primer 2: ACTCTCCACTGAATCACTGCCA (SEQ ID Nr. 12) und der Referenz (GAPDH-Primerl: ATCAACGGGAAGCCCATCA (SEQ ID Nr. 13), GAPDH-Primer2: GACATACTCAGCACCGGCCT (SEQ ID Nr. 14)) wurden anhand der erfindungsgemäß verwendeten Nukleinsäure und der bekannten Sequenz von GAPDH mit der Primer-Express-Software für Macintosh PPC Version 1.0 (PE Applied Biosystems, P/N 402089, 1998) ausgewählt. Für die PCR wurde das SYBR Green® PCR Core Reagents Kit (4304886, PE Applied Biosystems) verwendet. Die Konzentration der Primer in der PCR wurde zunächst im Bereich von 50 nM bis 600 nM optimiert und die Spezifität der PCR durch Analyse der Länge der amplifizierten Produkte in einer Agarose-Gel Elekrophorese geprüft. Anschließend wurde mittels einer Verdünnungsreihe die Effizienz der PCR-Systeme ermittelt (User Bulletin #2, Relative Quantitation of Gene Expression, PE Applied Biosystems, 1997). Dabei ergab sich, daß für beide cDNAs die Effizienz der Amplifikation bei 100% lag, d. h. bei jeder 1:2 Verdünnung der cDNA wurde ein Zyklus mehr benötigt, um den Fluoreszensschwellenwert zu überschreiten.

Für die Quantifizierung wurden je Ansatz cDNA aus 10 ng revers transkribierter Gesamt-RNA in einem Gesamtvolumen von 25 µl amplifiziert. Die Laufbedingungen für die PCR entsprachen den Angaben des Herstellers (PE Applied Biosystems, SYBR Green® PCR and RT-PCR Reagents Protocol, 1998). Die CT-Werte wurden analysiert und die Abundanz von *NM23-M2* relativ zu GAPDH wurde berechnet. Dabei bestätigte sich die leichte Induktion von *NM23* in gut heilenden Wunden und die starke Induktion in schlecht heilenden Wunden Dexamethason behandelter Tiere (Figur 2).

### Beispiel 3: Analyse des Expressionsmusters von NM23-H1 mittels "RNase Protection Assay"

Die Expression von NM23-H1 wurde mit Hilfe des "RNase Protection Assays" analysiert. Der Test wurde wie in der Literatur beschrieben durchgeführt (Sambrook et al., supra Kapitel 7, Seite 7.71 bis 7.78; Werner et al., 1992; Growth Factors and Receptors: A Practical Approach 175-197, Werner, 1998, Proc. Natl. Acad. Sci. USA 89: 6896-6900). Es wurde *in vitro* transkribierte und radioaktiv markierte Gegenstrang-RNA als Hybridisierungssonde eingesetzt. Ein 266 bp langes NM23-H1 Fragment (SEQ ID Nr. 15) wurde über glatte Enden in die EcoRV-Schnittstelle des pBluescript II KS Vektors (Stratagene) kloniert. Das Plasmid wurden vor der Transkription mit XbaI linearisiert (Länge des Transkripts ohne Vektorsequenz: 266 Basenpaare, Sequenz der Sonde SEQ ID Nr. 15). Die Transkriptionen wurde mit T₃-Polymerase (Roche Diagnostics, Mannheim) in Gegenwart von ³²P-UTP (35 µCi/Ansatz) (Amersham, Braunschweig) nach Angaben des Herstellers durchgeführt. Die Sonde wurde durch Gelelektrophorese und Elution aufgereinigt (Sambrook et al., supra, Kapitel 6, Seite 6.36 bis 6.48). Für die Hybridisierungsreaktion wurden je ca. 100.000 CPM des markierten Transkripts eingesetzt. 10 µg Gesamt-RNA, die mit Standardmethoden (Chomczynski und Sacchi, 1987, Anal. Biochem. 162: 156-159, Chomczynski und Mackey, 1995, Anal. Biochem. 225: 163-164) aus Haut- und Darmbiopsien isoliert wurde, wurden hierfür mit dem Transkript gefällt, in 10 µl Hybridisierungspuffer (80% entionisiertes Formamid, 400 mM NaCl, 40 mM Pipes pH 4,6, 1 mM EDTA) aufgenommen und über Nacht bei 42°C hybridisiert. Anschließend wurde ein RNase A/T1-Verdau (Boehringer, RNase A: 0,8 µg/Ansatz, RNase T1: 20 U/Ansatz) durchgeführt. Nach Inaktivierung der RNase durch Proteinase K-Verdau (Boehringer, 30 µg/Ansatz) und einer Phenolextraktion wurden die Proben mit Ethanol nach Standardmethoden (Sambrook et al., supra) präzipitiert. Die Proben wurden anschließend gelektrophoretisch auf einem denaturierendem 5% Acrylamidgel (7 M Harnstoff) aufgetrennt. Das Gel wurde getrocknet und die radioaktiven Signale mittels Autoradiografie nachgewiesen (Figuren 3 und 4).

In dem RNase Protection Assay mit RNAs, die aus Biopsien von 3 unterschiedlichen Psoriasis- und 4 unterschiedlichen Kontroll-Patienten isoliert wurde, konnte bei allen Psoriasisbiopsien eine erhöhte Expression von NM23-H1 in von Psoriasis befallenen im Vergleich zu Kontroll-Hautproben festgestellt werden (Figur 3). Der RNAse Protection Assay mit RNA aus Gewebeproben von 5 an Morbus Crohn erkrankten Personen zeigte eine vermehrte Expression von NM23-H1 in entzündlichen Darmabschnitten im Vergleich zu einem Kontrollprobanden (Figur 4).

### Beispiel 4: Analyse der Expression von NM23-M1 in Gewebeschnitten von Maus Wunden.

Die Expression von NM23-M1 in Wunden wurde durch in situ Hybridisierung analysiert. Der Test wurde wie in der Literatur beschrieben durchgeführt (Werner et al., 1992; Growth Factors and Receptors: A Practical Approach 175-197, Werner, 1998, Proc. Natl. Acad. Sci. USA 89: 6896-6900). Ein 256 bp langes NM23-M1 Fragment (SEQ ID Nr. 16) wurde über glatte Enden in die EcoRV-Schnittstelle des pBluescript II KS Vektors (Stratagene) kloniert. Mit diesem Vektor wurde wie beschrieben (Werner, 1998, supra) eine radioaktiv markierte Gegenstrang RNA als Hybridisierungssonde hergestellt und eingesetzt. Mit dieser Sonde wurden Hybridisierungen an Gefrierschnitten von 5 Tages-Wunden von Mäusen durchgeführt. Es zeigte sich, dass das Gen NM23-M1 verstärkt im hyperproliferativen Epithel am Rand des Wundgewebes exprimiert wird. Dagegen wurde in den gesunden Hautbereichen eine nur schwache bis nicht detektierbare Färbung beobachtet.

### Beispiel 5: Analyse des Expressionsmusters von NM23-M1 und NM23-M2 mRNAs während der Wundheilung der Maus mittels "TaqMan Analyse"

Die Kinetik der Regulation der Expression der NM23 mRNAs NM23-M1 und NM23-M2 während der normalen Wundheilung der adulten Maus wurde mittels "TaqMan Analyse" im GeneAmp5700 von Applied Biosystems untersucht. Normal heilende Wunden und intakte Haut wurde aus mit isotonischer Kochsalzlösung behandelten 10 Wochen alten, adulten BALB/c-Mäusen durch Scherenschnitt wie oben beschrieben gewonnen.

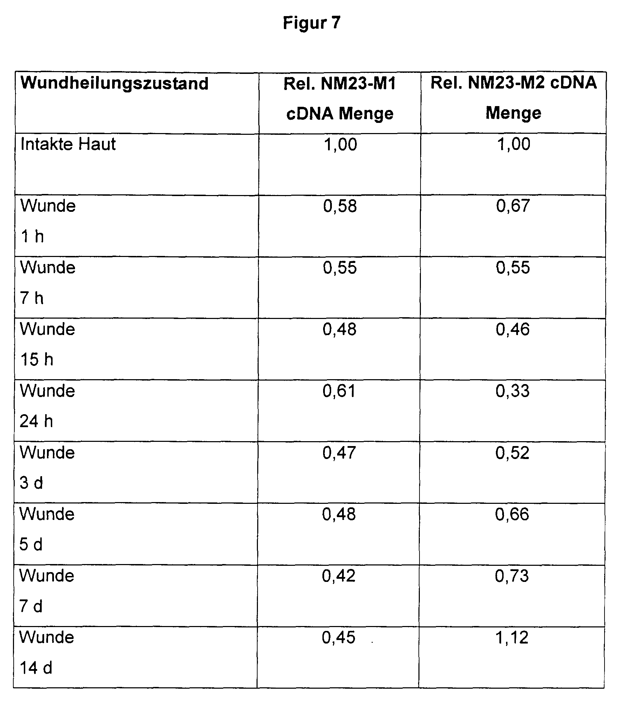

Die Isolierung der RNA erfolgte durch Homogenisieren der Biopsien in RNAc-lean Puffer (AGS, Heidelberg), dem 1/100 Volumenanteil 2-Mercaptoethanol zugesetzt worden war unter Verwendung eines Dispersers. Anschließend wurde die RNA durch zweimaliges Phenolisieren mittels mit Wasser gesättigtem saurem Phenol und in Gegenwart von 1-Brom-3-Chlor-Propan extrahiert. Anschließend wurden eine Isopropanol- und eine Ethanol-Fällung durchgeführt und die RNA mit 75% Ethanol gewaschen. Danach wurde ein DNase I-Verdau der RNA durchgeführt. Hierzu wurden 20 µg RNA (ad 50 µl mit DEPC-behandeltem Wasser) mit 5,7 µl Transkriptions-Puffer (Roche), 1 µl RNase-Inhibitor (Roche; 40 U/µl) und 1 µl DNase I (Roche; 10 U/µl) 20 min bei 37°C inkubiert. Dann wurde wiederum 1 µl DNase I zugegeben und weitere 20 min bei 37°C inkubiert. Anschließend wurde die RNA phenolisiert, Ethanol-gefällt und gewaschen. Alle oben aufgeführten Schritte wurden mit DEPC (Diethylpyrocarbonat)-behandelten Lösungen bzw. Flüssigkeiten durchgeführt soweit diese keine reaktiven Aminogruppen enthielten. Anschließend erfolgte die Herstellung von cDNA aus der extrahierten RNA. Dies erfolgte in Gegenwart von 1 x TaqMan RT-Puffer (Perkin Elmer), 5,5 mM MgCl₂ (Perkin Elmer), je 500 µM dNTPs (Perkin Elmer), 2,5 µM Random Hexamere (Perkin Elmer), 1,25 µl MultiScribe Reverse Transcriptase (50 U/µl Perkin Elmer), 0,4 µl RNase Inhibitor (20 U/µl, Perkin Elmer), 20 µl RNA (50 ng/µl) und DEPC-behandeltem Wasser (ad 100 µl Volumen). Nach Zugabe der RNA und guter Durchmischung wurde die Lösung auf 2 0,2 ml Gefäße verteilt (je 50 µl) und die reverse Transkription in einem Temperatur-Cycler durchgeführt (10 min bei 25°C; 30 min bei 48°C und 5 min bei 95°C). Die nachfolgende Quantifizierung der cDNA erfolgte mittels quantitativer PCR unter Verwendung des SYBR Green PCR Master Mixes (Perkin Elmer), wobei für jede zu bestimmende NM23 cDNA-Spezies eine Dreifachbestimmung (jeweils mit Target-Primern und GAPDH-Primern) durchgeführt wurde. Die Stammlösung für jedes Triplett enthielt bei 57 µl Gesamtvolumen 37,5 µl 2 x SYBR Master Mix, 0,75 µl AmpErase UNG (1 U/µl) und 18,75 µl DEPC-behandeltes Wasser. Pro Dreifachbestimmung wurden zu 57 µl Stammlösung je 1,5 µl Vorwärts- und Rüchwärts-Primer in einem zuvor optimierten Konzentrationsverhältnis hinzugefügt. Je 60 µl der Stammlösung/Primer-Mischung wurde mit 15 µl cDNA-Lösung (2 ng/µl) gemischt und auf 3 wells verteilt. Parallel dazu wurde eine Stammlösung mit Primern zur Bestimmung von GAPDH (SEQ ID Nr. 13 und SEQ ID Nr. 14) als Referenz hergestellt, mit weiteren 15 µl der gleichen cDNA-Lösung gemischt und auf 3 wells verteilt. Außerdem wurden um eine Standardkurve für die GAPDH-PCR zu erstellen verschiedene cDNA-Lösungen als Verdünnungsreihe hergestellt (4 ng/µl; 2 ng/µl; 1 ng/µl; 0,5 ng/µl und 0,25 ng/µl). Je 15 µl dieser cDNA-Löungen wurden mit 60 µl Stammlösung/Primer-Mischung zur Bestimmung von GAPDH gemischt und auf 3 wells verteilt. Ebenso wurde jeweils eine Standardkurve für die PCR der zu untersuchenden NM23-Homologe erstellt; dabei wurden dieselben Verdünnungen, die auch für die GAPDH-Standardkurve zum Einsatz kamen, verwendet. Als Kontrolle diente ein PCR Ansatz ohne cDNA. Zu jeweils 60 µl Stammlösung/Primer-Mischung von jeweils Target und GAPDH wurden je 15 µl DEPC-Wasser gegeben, gemischt und jeweils auf 3 wells verteilt. Die Amplifikation der Ansätze wurde im GeneAmp 5700 durchgeführt (2 min bei 50°C; 10 min bei 95°C, gefolgt von 3 Zyklen mit 15 sek bei 96°C und 2 min bei 60°C; danach 37 Zyklen mit 15 sek bei 95°C und 1 min bei 60°C). Die Auswertung erfolgte durch die Bestimmung der relativen Abundanz jedes NM23-Gens im Bezug auf die GAPDH-Referenz. Dazu wurde zuerst eine Standardkurve erstellt, indem die C_{T}-Werte der Verdünnungsreihe gegen den Logarithmus der cDNA-Menge im PCR-Ansatz (in ng umgeschriebener RNA) aufgetragen wurde und die Steigung (s) der Geraden ermittelt wurde. Die Effizienz (E) der PCR ergibt sich dann wie folgt: E = 10^{-1/s} ― 1. Die relative Abundanz (X) der untersuchten NM23 cDNA-Spezies (Y) im Bezug auf GAPDH ist dann: X=(1+E_{GAPDH})^{C}_{T}^{(GAPDH)}/(1+E_{Y})^{C}_{T}^{(Y)}. Anschließend wurden die Zahlenwerte normiert, indem die Menge an cDNA aus intakter Haut der 10 Wochen alten BALB/c Kontrolltiere gleich 1 gesetzt wurde. Die relativen Änderungen der NM23-M1 bzw. NM23-M2 Expression zu verschiedenen Zeitpunkten nach Verletzung adulter Mäuse sind in Figur 7 zusammengestellt.

So konnte unter Verwendung geeigneter Primer zum Nachweis von NM23-M1 (NM23-Primer 3: TCCTGGCACAGTCAGACAACA (SEQ ID Nr. 17); NM23-Primer 4: TTCACAACCTCACACATCCTCC (SEQ ID Nr. 18)) und NM23-M2 (NM23-Primer 1 (SEQ ID Nr. 11) und NM23-Primer 2 (SEQ ID Nr.12)) gezeigt werden, daß die Expression beider Homologe während der Wundheilung verringert ist (Figur 7). Bei NM23-M1 ist im Verlauf der Wundheilung eine praktisch gleichbleibende Reduktion der NM23 mRNA-Menge auf ca. 50 % der Menge in intakter Haut zu beobachten. Ein ähnliches Muster ist bei NM23-M2 nachweisbar: in diesem Fall ist eine deutliche Reduktion der Expression während der Wundheilung relativ zu intakter Haut zwischen 1 h und 7 d nach Wundsetzung meßbar. Erst zum Zeitpunkt 14 d nach Wundsetzung ist ein Wiederanstieg ungefähr auf den Ausgangswert in intakter Haut beobachtbar. Insgesamt weisen jedoch beide Homologe eine vergleichbar starke Verringerung der Expression während eines langen Zeitraums der Wundheilung auf. Dieses Ergebnis scheint zunächst im Gegensatz zu Beispiel 1 und 2 zu stehen, die eine schwache Steigerung der NM23 Expression in normal heilenden Wunden zeigt. Jedoch demonstriert das Ergebnis des Versuchs aus Beispiel 4, daß es nicht zu einem generellen Anstieg der NM23 Expression in der Wunde kommt. Vielmehr wurde eine verstärkte Färbung am hyperproliferativen Epithel festgestellt, während keine signifikante Expression in anderen Gewebeschichten beobachtet wurde. Dieses Ergebnis impliziert jedoch, daß wundheilungsabhängige Änderungen der mRNA-Menge bei komplexen Änderungen des räumlichen Expressionsmusters, und einer Empfindlichkeit der Nachweismethode "in situ Hybridisierung" die unter der der "real time RTPCR" liegt, in diesem Fall nur unzureichend beurteilt werden können: durch die komplexen räumlichen Änderungen der NM23 Expression (wie durch Beispiel 4 impliziert) können kleine Variabilitäten bei der Biopsienahme zu unterschiedlichen Ergebnissen bei der Bestimmung der wundheilungsabhängigen Änderung der NM23 mRNA Menge mittels "real time RTPCR" (Beispiel 2) und subtraktiver Hybridisierung (Beispiel 1) im Gegensatz zur Bestimmung mittels "TaqMan Analyse" (Beispiel 5) führen. Überraschenderweise konnte trotz dieser schwierigen Voraussetzungen NM23 als Genfamilie identifiziert und verifiziert werden.

### Beispiel 6: Differentielle Expression von NM23-H1 und NM23-H2 in humanen Wunden

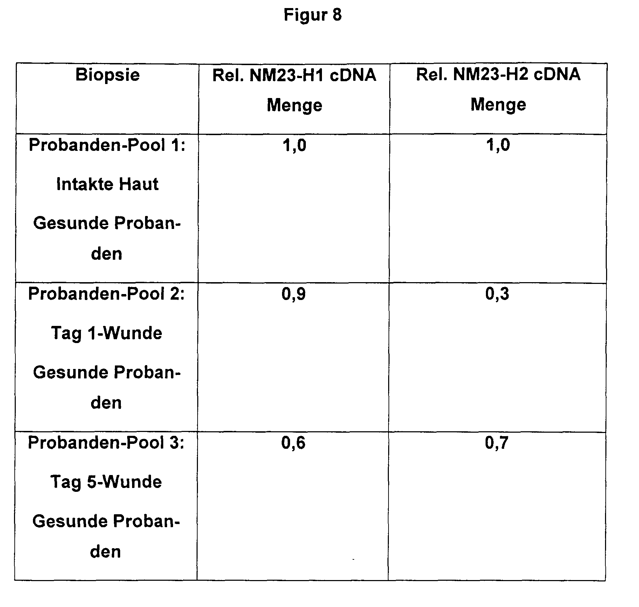

In Beispiel 3 wurde die differentielle Regulation von NM23-H1 in Psoriasis und Morbus Crohn Patienten untersucht. Es sollte nun anhand der normal heilenden Wunde untersucht werden, ob die in Beispiel 5 bestimmte differentielle Regulation der Expression von NM23-M1 und NM23-M2 sich auch beim Menschen beobachten läßt. Dazu wurden von gesunden Probanden aus unbehandelter intakter Haut, von Tag 1- Wunden oder von Tag 5-Wunden mittels 4 mm bzw. 6 mm Stanzen Hautproben entnommen. Von jeder Gruppe (intakte Haut, Tag 1-Wunde, Tag 5-Wunde) wurden die Biopsien von jeweils 14 Probanden gepoolt. Die Biopsien wurden mittels einer Kugelmühle zerkleinert und daraus, wie im Beispiel 5 beschrieben, RNA isoliert wie in Beispiel 5 beschrieben, danach DNase I verdaut und anschließend in cDNA umgeschrieben. Die Quantifizierung wundheilungsrelevanter cDNAs erfolgte ebenfalls wie in Beispiel 5 beschrieben. Die Ergebnisse der Experimente sind in Figur 8 zusammengestellt. Für die Analyse der NM23-Homologe wurden die Primer für die Amplifikation (hGAPDH-Primer 1: CATGGGTGTGAACCATGAGAAG (SEQ ID Nr. 25); hGAPDH-Primer 2: GCTAAGCAGTTGGTGGTGCA (SEQ ID Nr. 26), NM23-H1 Primer 1: GAAATTCATGCAAGCTTCCGA (SEQ ID Nr. 19), NM23-H1 Primer 2: CAGGTCAACGTAGTGTTCCTTGAG (SEQ ID Nr. 20); NM23-H2 Primer 1: CTGGTTGACTACAAGTCTTGTGCTC (SEQ ID Nr. 21); NM23-H2 Primer 2: TCCACCTCTTATTCATAGACCCAGT (SEQ ID Nr. 22)) anhand der bekannten Sequenzen von humanem GAPDH (GenBank: M17851) und humanem NM23-H1 und NM23-H2 (EMBL: X17620 und EMBL: X58965) ausgewählt. Für die Quantifizierung wurden je Ansatz cDNA aus 10 ng revers transkribierter Gesamt-RNA in einem Gesamtvolumen von 25 µl amplifiziert. Die PCR wurde entsprechend den Angaben des Herstellers (PE Applied Biosystems, SYBR Green PCR and RT-PCR Reagents Protocol, 1998) durchgeführt. Die C_{T}-Werte wurden ermittelt und daraus wurde die Häufigkeit von NM23-mRNA relativ zu GAPDH-mRNA berechnet. Die Ergebnisse des Versuchs sind in Figur 8 dargestellt. Es wurde beobachtet, daß beide Homologe eine leichte Verringerung der Expression in Wundgewebe zeigten. Im Gegensatz zu der Analyse der murinen Biopsien in Beispiel 5 wurden bei diesem Versuch zwei verschiedene Zeitpunkte ausgewählt. Die Ergebnisse mit humanem Gewebe zeigen eine signifikante Koinzidenz mit den Ergebnissen betreffend die Wundheilungskinetik der Maus (Beispiel 5): NM23-M1 und NM23-H1 sind zum Zeitpunkt 1 d nach Verwundung um 70 % schwächer in Biopsien exprimiert und werden beide zum Zeitpunkt 5 d nach Wundsetzung wieder leicht verstärkt exprimiert (66% bzw. 70 % des Ausgangswertes). Auch NM23-M2 und NM23-H2 werden in Tag-5 Wunden in vergleichbarer Weise schwächer exprimiert (48% vs. 60% des Ausgangswertes). Somit konnte bestätigt werden, daß die Regulation beider Gene bei der Wundheilung eine essentielle Rolle spielt und daß die Modulation der Menge mindestens eines Homologs, vorzugsweise beider Homologe zur Prävention und/oder Diagnose und/oder Behandlung von Erkrankungen von Hautzellen eingesetzt werden kann.

### Beispiel 7: Lokalisation von NM23-H2 in Biopsien von intakter Haut, normal heilender Wunde, Ulkus, sowie von nicht-läsionaler und läsionaler Psoriasis_Haut mittels in situ Hybridisierung

Für das Experiment wurde von einem gesunden Patienten Biopsien von intakter Haut als auch von einer normal heilenden Tag 5-Wunde wie in Beispiel 6 beschrieben entnommen. Außerdem wurden Biopsien von jeweils nicht-läsionaler und läsionaler Haut von 10 Psoriasis Patienten und von intakter Haut und der Wunde eines Ulkus-Patienten (Ulcera cruris venosum) wie oben beschrieben entnommen. Die Gewebeschnitte wurden in 4% Paraformaldehyd fixiert, mit Proteinase K (1µg/ml isotonischer Salzlösung) für 10 min bei 37°C behandelt, wiederum mit Paraformaldehyd und anschließend mit Acetanhydrid (0,5 ml in 0,1 M Triethanolamin, pH 8,0) behandelt.

Die Lokalisierung der mRNA von humanem NM23-H2 erfolgte durch radioaktive *in situ* Hybridisierung. Dazu wurden die Paraformaldehyd-fixierten Schnitte in Paraffin eingebettet. Die Herstellung der Hybridisierungssonde basierte auf der *in vitro* Transkription von einem partiellen NM23-H2 cDNA-Fragment in Gegenwart von α-³⁵S-UTP. Die Herstellung des PCR-Produkts erfolgte mit Primern, an die die Promotersequenzen für eine Transkription in sense und antisense Richtung angefügt wurden. (T3-NM23-H2-Primer: AAT TAA CCC TCA CTA AAG GGG GAG GGG CTG AAC GTG GTG AAG AC (sense-Kontrollprimer mit T3-Promotor; SEQ ID Nr. 23), Sp6-NM23-Primer: ATT TAG GTG ACA CTA TAG AAT ACA CGC CGT GCT GAA GGA GAC TGC (antisense-primer mit Sp6-Promotor; SEQ ID Nr. 24). Für die *in vitro* Transkription wurden 60 µCi ³⁵S-UTP und je 5 mM ATP, GTP und CTP, sowie entweder 25 U T3 oder T7 RNA Polymerase (Roche), 1 µg PCR-Produkt, 10 mM Dithiothreitol, 40 U RNAse Inhibitor (Roche) und 1 X TB-Puffer (Roche) verwendet.

Die humanen Gewebeschnitte (siehe oben) wurden auf Objektträgern mit Proteinase K-behandelt, mit Paraformaldehyd fixiert und anschließend acetyliert. Anschließend wurde die Schnitte in einer Kammer, in der feuchte, mit 50% Formamid/4x SSC getränkte Whatman Papiere lagen, mit 30 µl Hybridisierungslösung bedeckt und 2,5 h bei 60°C inkubiert. Danach wurden die Schnitte mit 0,7 x 10⁶ cpm radioaktiv markierter Ribosonde in 30 µl Hybridisierungslösung 16 h bei 60°C inkubiert. Anschließend wurden die Schnitte unter stringenten Bedingungen gewaschen, mit RNAse A inkubiert und mit Ethanol dehydratisiert. Die Schnitte wurden dann mit Photoemulsion (Kodak IBO 1433) überschichtet, unter Ausschluß von Licht und Sauerstoff für 2-6 Wochen bei 4°C gelagert und anschließend mittels Entwickler und Fixierer (Kodak IBO 1433) nach Angaben des Herstellers entwickelt.

In intakter Haut des gesunden Patienten und des Ulkus-Patienten, sowie in nicht-läsionaler Haut der Psoriasis Patienten wurde keine oder nur schwache Signale beobachtet. Im Gewebeschnitt der normal heilenden Tag-5 Wunde wurden dagegen Signale in den basalen Zellschichten des hyperproliferativen Epithels beobachtet. Dies deutet darauf hin, daß die Induktion der NM23-H2-Expression vor allem in derjenigen Zellschicht essentiell ist, die für die Schließung der Wunde durch Proliferation und Migration verantwortlich ist. Dies ist konsistent mit der Beobachtung, daß am Wundrand und im Wundgrund der nicht oder nur schlecht heilenden Ulkuswunde keine signifikante Färbung festgestellt wurde. Die wundspezifische Regulation der NM23-H2-Expression ist daher essentiell für den normalen Verlauf der Wundheilung. In läsionaler der Psoriasis Hautbiopsien wurde ebenfalls eine signifikant erhöhte Färbung in den basalen Zellschichten des hyperproliferativen Epithels gefunden im Vergleich zu intakter Haut des gesunden Probanden oder zur nicht-läsionaler Haut der Psoriasis Patienten. Dies ist konsistent mit dem Ergebnis des Versuchs aus Beispiel 3, bei dem gezeigt wurde, daß die Menge an NM23-H1 in psoriatischer Haut erhöht ist. Dieser Versuch verdeutlicht, daß die Regulation der NM23-Expression für eine intakte, gesunde Haut als auch einen normalen Wundheilungsprozeß essentiell ist und daß eine Fehlregulation der Expression zu Erkrankungen von Hautzellen, wie zum Beispiel einer verzögerten Wundheilung oder Psoriasis führen kann und NM23, vorzugsweise beide Homologe zur Prävention und/oder Diagnose und/oder Behandlung von Erkrankungen von Hautzellen eingesetzt werden kann. Schlecht heilende Wunden sind mit verminderter NM23-Menge assoziiert, während Psoriasis-Patienten, deren Keratinozyten durch krankhafte Proliferation gekennzeichnet sind, verstärkte NM23-Expression aufweisen.

Daher ist zur Behandlung von Hautzellen die Expression oder Aktivität von NM23 zu modulieren; bevorzugterweise ist die Expression oder Aktivität von NM23 zu aktivieren im Falle krankhaft gestörter Wundheilung und sie ist bevorzugterweise zu inhibieren im Falle einer hyperproliferativen Störung von Hautzellen, insbesondere Psoriasis.

## Patentansprüche

1. Verwendung mindestens eines Polypeptids der Genfamilie NM23 gemäß einer der SEQ ID Nr. 1 bis SEQ ID Nr. 10 oder einer funktionellen Variante davon oder mindestens einer für oben genannte Polypeptide kodierenden Nukleinsäure oder einer Variante davon zur Analyse und/oder Diagnose und/oder Prävention und/oder Behandlung von Haut- und/oder Darmerkrankungen und/oder von Wundheilung und/oder deren krankhaften Störungen.

2. Verwendung mindestens eines Polypeptids der Genfamilie NM23 gemäß einer der SEQ ID Nr. 1 bis SEQ ID Nr. 10 oder einer funktionellen Variante davon oder mindestens einer für oben genannte Polypeptide kodierenden Nukleinsäure oder einer Variante davon zur Identifizierung von mindestens einer pharmakologisch aktiven Substanz, die im Zusammenhang zu Haut- und/oder Darmerkrankungen und/oder mit der Wundheilung und/oder deren krankhaften Störungen steht.

3. Verwendung nach Anspruch 2, **dadurch gekennzeichnet**, daß die pharmakologisch aktive Substanz ausgewählt ist aus einer Nukleinsäure, beispielsweise in Form einer DNA-Bindungsdomäne, eines Promotors oder eines Enhancers, eines Repressors, oder einem Polypeptid, beispielsweise in Form eines Aktivators oder eines Inhibitors.

4. Verwendung einer Nukleinsäure nach einem der Ansprüche 1 bis 3, **dadurch gekennzeichnet**, daß die Nukleinsäure eine DNA oder RNA, vorzugsweise eine DNA, insbesondere eine doppelsträngige DNA ist.

5. Verwendung einer Nukleinsäure nach einem der Ansprüche 1 bis 4, **dadurch gekennzeichnet**, daß die Nukleinsäuresequenz mindestens ein Intron und/oder eine polyA-Sequenz aufweist.

6. Verwendung einer Nukleinsäure nach einem der Ansprüche 1 bis 5 in Form ihrer antisense-Sequenz.

7. Verwendung einer Nukleinsäure nach einem der Ansprüche 1 bis 6, **dadurch gekennzeichnet**, daß die Nukleinsäure synthetisch hergestellt worden ist.

8. Verwendung eines Polypeptids nach einem der Ansprüche 1 bis 3, **dadurch gekennzeichnet**, daß das Polypeptid synthetisch hergestellt worden ist.

9. Verwendung eines Polypeptids nach einem der Anspruch 1 bis 3 oder 8 **dadurch gekennzeichnet**, daß das Polypeptid ein Fusionsprotein ist.

10. Verwendung eines Vektors, vorzugsweise in Form eines Plasmids, Shuttle Vektors, Phagemids, Cosmids, der mindestens eine Nukleinsäure, die für Polypeptide der Genfamilie NM23 gemäß einer der SEQ ID Nr. 1 bis SEQ ID Nr. 10 kodiert, oder eine Variante davon enthält, zur Analyse und/oder Diagnose und/oder Prävention und/oder Behandlung von Haut- und/oder Darmerkrankungen und/oder von Wundheilung und/oder deren krankhaften Störungen.

11. Verwendung nach Anspruch 10, **dadurch gekennzeichnet**, daß der Vektor ein Expressionsvektor ist.

12. Verwendung nach Anspruch 10, **dadurch gekennzeichnet**, daß der Vektor ein knock-out Genkonstrukt ist.

13. Verwendung nach einem der Ansprüche 10 bis 12, **dadurch gekennzeichnet**, daß der Vektor ein gentherapeutisch wirksamer Vektor ist

14. Verwendung einer Wirtszelle, die mindestens eine Nukleinsäure, die für Polypeptide der Genfamilie NM23 gemäß einer der SEQ ID Nr. 1 bis SEQ ID Nr. 10 kodiert oder eine Variante davon enthält, zur Analyse und/oder Diagnose und/oder Prävention und/oder Behandlung von Haut- und/oder Darmerkrankungen und/oder von Wundheilung und/oder deren krankhaften Störungen.

15. Verwendung einer Wirtszelle nach Anspruch 14, **dadurch gekennzeichnet**, daß die Nukleinsäure in Form eines Vektors gemäß mindestens einem der Ansprüche 10 bis 13 in die Zelle eingefügt wird.

16. Verwendung einer Wirtszelle nach Anspruch 14 oder 15, **dadurch gekennzeichnet**, daß es sich um eine Hautzelle oder Darmzelle handelt.

17. Verwendung einer Wirtszelle nach Anspruch 14 oder 15, **dadurch gekennzeichnet**, daß die Wirtszelle eine transgene embryonale nichtmenschliche Stammzelle ist.

18. Verwendung eines transgenen nichtmenschlichen Säugetiers, das eine transgene embryonalen nichtmenschlichen Stammzelle gemäß Anspruch 17 enthält **dadurch gekennzeichnet**, daß das transgene nichtmenschlichen Säugetier zur Analyse und/oder Diagnose von Haut- und/oder Darmerkrankungen und/oder von Wundheilung und/oder deren krankhaften Störungen verwendet wird.

19. Verwendung nach Anspruch 18, **dadurch gekennzeichnet**, daß das Genom des transgenen nichtmenschlichen Säugetiers eine Expressionskassette oder ein knock-out Genkonstrukt nach Anspruch 11 oder 12 enthält.

20. Verwendung eines Antikörpers zur Analyse und/oder Diagnose und/oder Prävention und/oder Behandlung von Haut- und/oder Darmerkrankungen und/oder von Wundheilung und/oder deren krankhaften Störungen und/oder zur Identifizierung von pharmakologisch aktiven Substanzen, **dadurch gekennzeichnet**, daß der Antikörper gegen ein Polypeptid aus der Genfamilie NM23 gemäß einer der SEQ ID Nr. 1 bis SEQ ID Nr. 10 oder gegen eine funktionelle Variante davon gerichtet ist.

21. Verwendung eines Diagnostikums zur Diagnose von Haut- und/oder Darmerkrankungen und/oder von Wundheilung und/oder deren krankhaften Störungen, **dadurch gekennzeichnet**, daß das Diagnostikum mindestens ein Polypeptid der Genfamilie NM23 gemäß einer der SEQ ID Nr. 1 bis SEQ ID Nr. 10 oder eine funktionelle Variante davon oder mindestens eine für oben genannte Polypeptide kodierende Nukleinsäure oder eine Variante davon oder mindestens einen Antikörper gemäß Anspruch 20, gegebenenfalls zusammen mit geeigneten Zusatz- und/oder Hilfsstoffen, enthält.

22. Verwendung nach Anspruch 21, **dadurch gekennzeichnet**, daß das Diagnostikum eine Sonde, vorzugsweise eine DNA-Sonde, enthält.

23. Verwendung von mindestens einem Polypeptid der Genfamilie NM23 gemäß einer der SEQ ID Nr. 1 bis SEQ ID Nr. 10 oder einer funktionellen Variante davon oder mindestens einer für oben genannte Polypeptide kodierenden Nukleinsäure oder einer Variante davon oder mindestens eines Antikörpers nach Anspruch 20, welche(r) zusammen mit geeigneten Zusatz- und/oder Hilfsstoffen kombiniert wird, zur Herstellung eines Arzneimittels zur Behandlung von Haut- oder Darmerkrankungen und/oder von Wundheilung und/oder deren krankhaften Störungen.

24. Verwendung eines Tests zur Identifizierung funktioneller Interaktoren im Zusammenhang mit Haut- oder Darmerkrankungen und/oder mit der Wundheilung und/oder deren krankhaften Störungen, **dadurch gekennzeichnet**, daß er mindestens ein Polypeptid der Genfamilie NM23 gemäß einer der SEQ ID Nr. 1 bis SEQ ID Nr. 10 oder eine funktionelle Variante davon oder mindestens eine für oben genannte Polypeptide kodierende Nukleinsäure oder eine Variante davon oder mindestens einen Antikörper gemäß Anspruch 20, gegebenenfalls zusammen mit geeigneten Zusatz- und/oder Hilfsstoffen, enthält.

25. Verwendung eines auf einem Trägermaterial fixierten Arrays zur Analyse von Haut- und/oder Darmerkrankungen und/oder von Wundheilung und/oder deren krankhaften Störungen, **dadurch gekennzeichnet**, daß es mindestens ein Polypeptid der Genfamilie NM23 gemäß einer der SEQ ID Nr. 1 bis SEQ ID Nr. 10 oder eine funktionelle Variante davon oder mindestens eine für oben genannte Polypeptide kodierende Nukleinsäure oder eine Variante davon oder mindestens einen Antikörper nach Anspruch 20 enthält.

26. Verwendung nach Anspruch 25, **dadurch gekennzeichnet**, daß das Array ein DNA-Chip und/oder Protein-Chip ist.


SEQUENZPROTOKOLL

<110> Switch Biotech AG
      ETH Zürich

<120> Verwendung von Polypeptiden oder diese kodierende
      Nukleinsäuren der Genfamilie NM23 zur Diagnose oder
      Behandlung von Haut- und Darmerkrankungen sowie ihre
      Verwendung zur Identifizierung von pharmakologisch
      aktiven Substanzen

<130> S32775EP

<140>
<141>

<150> DE 100 08 330.7-41
<151> 2000-02-23

<150> US 60/199,312
<151> 2000-04-24

<160> 26

<170> PatentIn Ver. 2.1

<210> 1
<211> 152
<212> PRT
<213> Homo sapiens

<400> 1
Met Ala Asn Cys Glu Arg Thr Phe Ile Ala Ile Lys Pro Asp Gly Val
  1               5                  10                  15

Gln Arg Gly Leu Val Gly Glu Ile Ile Lys Arg Phe Glu Gln Lys Gly

            20                  25                  30

Phe Arg Leu Val Gly Leu Lys Phe Met Gln Ala Ser Glu Asp Leu Leu
        35                  40                  45

Lys Glu His Tyr Val Asp Leu Lys Asp Arg Pro Phe Phe Ala Gly Leu
    50                  55                  60

Val Lys Tyr Met His Ser Gly Pro Val Val Ala Met Val Trp Glu Gly
65                  70                  75                  80

Leu Asn Val Val Lys Thr Gly Arg Val Met Leu Gly Glu Thr Asn Pro
                85                  90                  95

Ala Asp Ser Lys Pro Gly Thr Ile Arg Gly Asp Phe Cys Ile Gln Val
            100                 105                 110

Gly Arg Asn Ile Ile His Gly Ser Asp Ser Val Glu Ser Ala Glu Lys
        115                 120                 125

Glu Ile Gly Leu Trp Phe His Pro Glu Glu Leu Val Asp Tyr Thr Ser
    130                 135                 140

Cys Ala Gln Asn Trp Ile Tyr Glu
145                 150

<210> 2
<211> 152
<212> PRT
<213> Mus musculus

<400> 2
Met Ala Asn Ser Glu Arg Thr Phe Ile Ala Ile Lys Pro Asp Gly Val
1               5                   10                  15

Gln Arg Gly Leu Val Gly Glu Ile Ile Lys Arg Phe Glu Gln Lys Gly
            20                  25                  30

Phe Arg Leu Val Gly Leu Lys Phe Leu Gln Ala Ser Glu Asp Leu Leu
        35                  40                  45

Lys Glu His Tyr Thr Asp Leu Lys Asp Arg Pro Phe Phe Thr Gly Leu
    50                  55                  60

Val Lys Tyr Met His Ser Gly Pro Val Val Ala Met Val Trp Glu Gly
65                  70                  75                  80

Leu Asn Val Val Lys Thr Gly Arg Val Met Leu Gly Glu Thr Asn Pro
                85                  90                  95

Ala Asp Ser Lys Pro Gly Thr Ile Arg Gly Asp Phe Cys Ile Gln Val
            100                 105                 110

Gly Arg Asn Ile Ile His Gly Ser Asp Ser Val Lys Ser Ala Glu Lys
        115                 120                 125

Glu Ile Ser Leu Trp Phe Gln Pro Glu Glu Leu Val Glu Tyr Lys Ser
    130                 135                 140

Cys Ala Gln Asn Trp Ile Tyr Glu
145                 150

<210> 3
<211> 152
<212> PRT
<213> Homo sapiens

<400> 3

Met Ala Asn Leu Glu Arg Thr Phe Ile Ala Ile Lys Pro Asp Gly Val
1               5                   10                  15

Gln Arg Gly Leu Val Gly Glu Ile Ile Lys Arg Phe Glu Gln Lys Gly
            20                  25                  30

Phe Arg Leu Val Ala Met Lys Phe Leu Arg Ala Ser Glu Glu His Leu
        35                  40                  45

Lys Gln His Tyr Ile Asp Leu Lys Asp Arg Pro Phe Phe Pro Gly Leu
    50                  55                  60

Val Lys Tyr Met Asn Ser Gly Pro Val Val Ala Met Val Trp Glu Gly
65                  70                  75                  80

Leu Asn Val Val Lys Thr Gly Arg Val Met Leu Gly Glu Thr Asn Pro
                85                  90                  95

Ala Asp Ser Lys Pro Gly Thr Ile Arg Gly Asp Phe Cys Ile Gln Val
            100                 105                 110

Gly Arg Asn Ile Ile His Gly Ser Asp Ser Val Lys Ser Ala Glu Lys
        115                 120                 125

Glu Ile Ser Leu Trp Phe Lys Pro Glu Glu Leu Val Asp Tyr Lys Ser
    130                 135                 140

Cys Ala His Asp Trp Val Tyr Glu
145                 150

<210> 4
<211> 152
<212> PRT
<213> Mus musculus

<400> 4

Met Ala Asn Leu Glu Arg Thr Phe Ile Ala Ile Lys Pro Asp Gly Val
1               5                   10                  15

Gln Arg Gly Leu Val Gly Glu Ile Ile Lys Arg Phe Glu Gln Lys Gly
            20                  25                  30

Phe Arg Leu Val Ala Met Lys Phe Leu Arg Ala Ser Glu Glu His Leu
        35                  40                  45

Lys Gln His Tyr Ile Asp Leu Lys Asp Arg Pro Phe Phe Pro Gly Leu
    50                  55                  60

Val Lys Tyr Met Asn Ser Gly Pro Val Val Ala Met Val Trp Glu Gly
65                  70                  75                  80

Leu Asn Val Val Lys Thr Gly Arg Val Met Leu Gly Glu Thr Asn Pro
                85                  90                  95

Ala Asp Ser Lys Pro Gly Thr Ile Arg Gly Asp Phe Cys Ile Gln Val
            100                 105                 110

Gly Arg Asn Ile Ile His Gly Ser Asp Ser Val Glu Ser Ala Glu Lys
        115                 120                 125

Glu Ile His Leu Trp Phe Lys Pro Glu Glu Leu Ile Asp Tyr Lys Ser
    130                 135                 140

Cys Ala His Asp Trp Val Tyr Glu
145                 150

<210> 5
<211> 187

<212> PRT

<213> Homo sapiens

<400> 5

Met Gly Gly Leu Phe Trp Arg Ser Ala Leu Arg Gly Leu Arg Cys Gly
1               5                   10                  15

Pro Arg Ala Pro Gly Pro Ser Leu Leu Val Arg His Gly Ser Gly Gly
            20                  25                  30

Pro Ser Trp Thr Arg Glu Arg Thr Leu Val Ala Val Lys Pro Asp Gly
        35                  40                  45

Val Gln Arg Arg Leu Val Gly Asp Val Ile Gln Arg Phe Glu Arg Arg
    50                  55                  60

Gly Phe Thr Leu Val Gly Met Lys Met Leu Gln Ala Pro Glu Ser Val
65                  70                  75                  80

Leu Ala Glu His Tyr Gln Asp Leu Arg Arg Lys Pro Phe Tyr Pro Ala
                85                  90                  95

Leu Ile Arg Tyr Met Ser Ser Gly Pro Val Val Ala Met Val Trp Glu
            100                 105                 110

Gly Tyr Asn Val Val Arg Ala Ser Arg Ala Met Ile Gly His Thr Asp
        115                 120                 125

Ser Ala Glu Ala Ala Pro Gly Thr Ile Arg Gly Asp Phe Ser Val His
    130                 135                 140

Ile Ser Arg Asn Val Ile His Ala Ser Asp Ser Val Glu Gly Ala Gln
145                 150                 155                 160

Arg Glu Ile Gln Leu Trp Phe Gln Ser Ser Glu Leu Val Ser Trp Ala
                165                 170                 175

Asp Gly Gly Gln His Ser Ser Ile His Pro Ala
            180                 185

<210> 6
<211> 168
<212> PRT
<213> Homo sapiens

<400> 6
Met Ile Cys Leu Val Leu Thr Ile Phe Ala Asn Leu Phe Pro Ala Ala
1               5                   10                  15

Cys Thr Gly Ala His Glu Arg Thr Phe Leu Ala Val Lys Pro Asp Gly
            20                  25                  30

Val Gln Arg Arg Leu Val Gly Glu Ile Val Arg Arg Phe Glu Arg Lys
        35                  40                  45

Gly Phe Lys Leu Val Ala Leu Lys Leu Val Gln Ser Ser Glu Glu Leu
    50                  55                  60

Leu Arg Glu His Tyr Ala Glu Leu Arg Glu Arg Pro Phe Tyr Gly Arg
65                  70                  75                  80

Leu Val Lys Tyr Met Ala Ser Gly Pro Val Val Ala Met Val Trp Gln
                85                  90                  95

Gly Leu Asp Val Val Arg Thr Ser Arg Ala Leu Ile Gly Ala Thr Asn
            100                 105                 110

Pro Ala Asp Ala Pro Pro Gly Thr Ile Arg Gly Asp Phe Cys Ile Glu
        115                 120                 125

Val Gly Asn Leu Ile His Gly Ser Asp Ser Val Glu Ser Ala Arg Arg
    130                 135                 140

Glu Ile Ala Leu Trp Phe Arg Ala Asp Glu Leu Leu Cys Trp Glu Asp
145                 150                 155                 160

Ser Ala Gly His Trp Leu Tyr Glu
                165

<210> 7
<211> 212
<212> PRT
<213> Homo sapiens

<400> 7
Met Glu Ile Ser Met Pro Pro Pro Gln Ile Tyr Val Glu Lys Thr Leu
  1               5                  10                  15

Ala Ile Ile Lys Pro Asp Ile Val Asp Lys Glu Glu Glu Ile Gln Asp
             20                  25                  30

Ile Ile Leu Arg Ser Gly Phe Thr Ile Val Gln Arg Arg Lys Leu Arg
         35                  40                  45

Leu Ser Pro Glu Gln Cys Ser Asn Phe Tyr Val Glu Lys Tyr Gly Lys
     50                  55                  60

Met Phe Phe Pro Asn Leu Thr Ala Tyr Met Ser Ser Gly Pro Leu Val
 65                  70                  75                  80

Ala Met Ile Leu Ala Arg His Lys Ala Ile Ser Tyr Trp Leu Glu Leu
                 85                  90                  95

Leu Gly Pro Asn Asn Ser Leu Val Ala Lys Glu Thr His Pro Asp Ser
            100                 105                 110

Leu Arg Ala Ile Tyr Gly Thr Asp Asp Leu Arg Asn Ala Leu His Gly
        115                 120                 125

Ser Asn Asp Phe Ala Ala Ala Glu Arg Glu Ile Arg Phe Met Phe Pro
    130                 135                 140

Glu Val Ile Val Glu Pro Ile Pro Ile Gly Gln Ala Ala Lys Asp Tyr
145                 150                 155                 160

Leu Asn Leu His Ile Met Pro Thr Leu Leu Glu Gly Leu Thr Glu Leu
                165                 170                 175

Cys Lys Gln Lys Pro Ala Asp Pro Leu Ile Trp Leu Ala Asp Trp Leu
            180                 185                 190

Leu Lys Asn Asn Pro Asn Lys Pro Lys Leu Cys His His Pro Ile Val
        195                 200                 205

Glu Glu Pro Tyr
    210

<210> 8
<211> 194
<212> PRT
<213> Homo sapiens

<400> 8
Met Thr Gln Asn Leu Gly Ser Glu Met Ala Ser Ile Leu Arg Ser Pro
1               5                   10                  15

Gln Ala Leu Gln Leu Thr Leu Ala Leu Ile Lys Pro Asp Ala Val Ala
            20                  25                  30

His Pro Leu Ile Leu Glu Ala Val His Gln Gln Ile Leu Ser Asn Lys
        35                  40                  45

Phe Leu Ile Val Arg Met Arg Glu Leu Leu Trp Arg Lys Glu Asp Cys
    50                  55                  60

Gln Arg Phe Tyr Arg Glu His Glu Gly Arg Phe Phe Tyr Gln Arg Leu
65                  70                  75                  80

Val Glu Phe Met Ala Ser Gly Pro Ile Arg Ala Tyr Ile Leu Ala His
                85                  90                  95

Lys Asp Ala Ile Gln Leu Trp Arg Thr Leu Met Gly Pro Thr Arg Val
            100                 105                 110

Phe Arg Ala Arg His Val Ala Pro Asp Ser Ile Arg Gly Ser Phe Gly
        115                 120                 125

Leu Thr Asp Thr Arg Asn Thr Thr His Gly Ser Asp Ser Val Val Ser
    130                 135                 140

Ala Ser Arg Glu Ile Ala Ala Phe Phe Pro Asp Phe Ser Glu Gln Arg
145                 150                 155                 160

Trp Tyr Glu Glu Glu Glu Pro Gln Leu Arg Cys Gly Pro Val Cys Tyr
                165                 170                 175

Ser Pro Glu Gly Gly Val His Tyr Val Ala Gly Thr Gly Gly Leu Gly
            180                 185                 190

Pro Ala

<210> 9

<211> 189

<212> PRT

<213> Mus musculus

<400> 9

Met Thr Ser Ile Leu Arg Ser Pro Gln Ala Leu Gln Leu Thr Leu Ala
1               5                   10                  15

Leu Ile Lys Pro Asp Ala Val Ala His Pro Leu Ile Leu Glu Ala Val
            20                  25                  30

His Gln Gln Ile Leu Ser Asn Lys Phe Leu Ile Val Arg Thr Arg Glu
        35                  40                  45

Leu Gln Trp Lys Leu Glu Asp Cys Arg Arg Phe Tyr Arg Glu His Glu
    50                  55                  60

Gly Arg Phe Phe Tyr Gln Arg Leu Val Glu Phe Met Thr Ser Gly Pro
65                  70                  75                  80

Ile Arg Ala Tyr Ile Leu Ala His Lys Asp Ala Ile Gln Leu Trp Arg
                85                  90                  95

Thr Leu Met Gly Pro Thr Arg Val Phe Arg Ala Arg Tyr Ile Ala Pro
            100                 105                 110

Asp Ser Ile Arg Gly Ser Leu Gly Leu Thr Asp Thr Arg Asn Thr Thr
        115                 120                 125

His Gly Ser Asp Ser Val Val Ser Ala Ser Arg Glu Ile Ala Ala Phe
    130                 135                 140

Phe Pro Asp Phe Ser Glu Gln Arg Trp Tyr Glu Glu Glu Glu Pro Gln

145                         150                         155                         160

Leu Arg Cys Gly Pro Val His Tyr Ser Pro Glu Glu Gly Ile His Cys
                    165                         170                         175

Ala Ala Glu Thr Gly Gly His Lys Gln Pro Asn Lys Thr
                180                         185

<210> 10
<211> 137
<212> PRT
<213> Homo sapiens

<400> 10
Met Gln Cys Gly Leu Val Gly Lys Ile Ile Lys Arg Phe Glu Gln Lys
 1               5                   10                  15

Gly Phe Arg Leu Val Ala Met Lys Phe Leu Pro Ala Ser Glu Glu His
                20                  25                  30

Leu Lys Gln His Tyr Ile Asp Leu Lys Asp Arg Pro Phe Phe Pro Gly
            35                  40                  45

Leu Val Lys Tyr Met Asn Ser Gly Pro Val Val Ala Met Val Trp Glu
    50                  55                  60

Gly Leu Asn Val Val Lys Thr Gly Arg Val Met Leu Gly Glu Thr Asn
65                  70                  75                  80

Pro Ala Asp Ser Lys Pro Gly Thr Ile Arg Gly Asp Phe Cys Ile Gln
                85                  90                  95

Val Gly Arg Asn Ile Ile His Gly Ser Asp Ser Val Lys Ser Ala Glu
            100                 105                 110

Lys Glu Ile Ser Leu Arg Phe Lys Pro Glu Glu Leu Val Asp Tyr Lys
        115                 120                 125

Ser Cys Ala His Asp Trp Val Tyr Glu
    130                 135

<210> 11
<211> 20
<212> DNA
<213> Mus musculus

<400> 11
ttcaaaacca ggcaccatcc                                                20

<210> 12
<211> 22
<212> DNA
<213> Mus musculus

<400> 12
actctccact gaatcactgc ca                                             22

<210> 13
<211> 19
<212> DNA
<213> Mus musculus

<400> 13
atcaacggga agcccatca                                                 19

<210> 14

<211> 20

<212> DNA

<213> Mus musculus

<400> 14

gacatactca gcaccggcct                                                    20

<210> 15

<211> 266

<212> DNA

<213> Homo sapiens

<400> 15

tctgaaattc atgcaagctt ccgaagatct tctcaaggaa cactacgttg acctgaagga 60

ccgtccattc tttgccggcc tggtgaaata catgcactca gggccggtag ttgccatggt 120

ctgggagggg ctgaatgtgg tgaagacggg ccgagtcatg ctcggggaga ccaaccctgc 180

agactccaag cctgggacca tccgtggaga cttctgcata caagttggca ggaacattat 240

acatggcagt gattctgtgg agagtg                                             266

<210> 16

<211> 256

<212> DNA

<213> Mus musculus

<400> 16

ctgaagtttc tgcaggcttc agaggacctt ctcaaggagc actacactga cctgaaggac 60

cgccccttct ttactggcct ggtgaaatac atgcactcag gaccagtggt tgctatggtc 120

tgggagggtc tgaatgtggt gaagacaggc cgcgtgatgc ttggagagac caaccccgca 180

gactctaagc ctgggaccat acgaggagac ttctgcatcc aagttggcag gaacatcatt 240

catggcagcg attctg                                                        256

<210> 17

<211> 21

<212> DNA

<213> Mus musculus

<400> 17

TCCTGGCACA GTCAGACAAC A 21

<210> 18

<211> 22

<212> DNA

<213> Mus musculus

<400> 18

TTCACAACCT CACACATCCT CC 22

<210> 19

<211> 21

<212> DNA

<213> Homo sapiens

<400> 19

GAAATTCATG CAAGCTTCCG A 21

<210> 20

<211> 24

<212> DNA

<213> Homo sapiens

<400> 20

CAGGTCAACG TAGTGTTCCT TGAG 24

<210> 21

<211> 25
<212> DNA
<213> Homo sapiens

<400> 21
CTGGTTGACT ACAAGTCTTG TGCTC 25

<210> 22
<211> 25
<212> DNA
<213> Homo sapiens

<400> 22
TCCACCTCTT ATTCATAGAC CCAGT 25

<210> 23
<211> 44
<212> DNA
<213> artificial sequence

<220>
<223> sense NM23H2-primer with T3 promoter sequence for PCR cloning and
/or the construction of a hybridization probe

<400> 23
AATTAACCCT CACTAAAGGG GGAGGGGCTG AACGTGGTGA AGAC 44

<210> 24
<211> 45
<212> DNA
<213> artificial sequence

<220>

<223> antisense NM23-primer with SP6 promoter sequence for PCR cloning
and/or construction of a hybridization probe

<400> 24
ATTTAGGTGA CACTATAGAA TACACGCCGT GCTGAAGGAG ACTGC 45

<210> 25
<211> 23
<212> DNA
<213> Homo sapiens

<400> 25
CATGGGTGTG AACCATGAG AAG 23

<210> 26
<211> 20
<212> DNA
<213> Homo sapiens

<400> 26
GCTAAGCAGT TGGTGGTGCA 20